# EUROPEAN PATENT SPECIFICATION

(11) **EP 2 909 626 B1**
(45) Date of publication and mention of the grant of the patent: **04.07.2018**
(21) Application number: 13776836.2
(22) Date of filing: 16.10.2013
(51) Int. Cl.: G01N 33/50

(54) **MEANS AND METHODS FOR DETERMINING A CLEARANCE NORMALIZED AMOUNT OF A METABOLITE DISEASE BIOMARKER IN A SAMPLE**
MITTEL UND VERFAHREN ZUR BESTIMMUNG EINER CLEARANCE-NORMALISIERTEN MENGE EINES STOFFWECHSELKRANKHEITBIOMARKERS IN EINER PROBE
SUPPORTS ET PROCÉDÉS PERMETTANT DE DÉTERMINER UNE QUANTITÉ NORMALISÉE DE DÉGAGEMENT D'UN BIOMARQUEUR DE LA MALADIE DE MÉTABOLITE DANS UN ÉCHANTILLON

(30) Priority: 18.10.2012 EP 12189027; 18.10.2012 US 201261715338 P
(43) Date of publication of application: 26.08.2015
(73) Proprietor: metanomics GmbH, 10589 Berlin (DE); Ruprecht-Karls-Universität Heidelberg, 69117 Heidelberg (DE)
(72) Inventor: FUHRMANN, Jens, 10115 Berlin (DE); FISCHER, Jenny, 10585 Berlin (DE); RESZKA, Regina, 16341 Panketal (DE); KATUS, Hugo A., 69120 Heidelberg (DE); FREY, Norbert, 24119 Kronshagen (DE); SIGL, Johanna, 68199 Mannheim (DE); WEIS, Tanja, 69257 Wiesenbach (DE)
(74) Representative: Herzog, Fiesser & Partner Patentanwälte PartG mbB
(86) International application number: PCT/EP2013/071648
(87) International publication number: WO 2014/060486

(56) References cited:
- WO-A2-01/01150
- BADGWELL ET AL: "Urinary mesothelin provides greater sensitivity for early stage ovarian cancer than serum mesothelin, urinary hCG free beta subunit and urinary hCG beta core fragment", GYNECOLOGIC ONCOLOGY, ACADEMIC PRESS, LONDON, GB, vol. 106, no. 3, 27 August 2007 (2007-08-27), pages 490-497, XP022215093, ISSN: 0090-8258, DOI: 10.1016/J.YGYNO.2007.04.022
- JERNBERG TOMAS ET AL: "Cystatin C - A novel predictor of outcome in suspected or confirmed non-ST-elevation acute coronary syndrome", CIRCULATION, vol. 110, no. 16, 19 October 2004 (2004-10-19), pages 2342-2348, XP002696858, ISSN: 0009-7322

## Description

The present invention relates to a method for determining a clearance normalized amount of a metabolite disease biomarker in a sample comprising the steps of (a) determining the amount of the disease biomarker in at least a first type of sample of a subject suspected to suffer from the disease, (b) determining the amount of a kidney function biomarker which correlates with the glomerular filtration rate (GFR) in the said at least first type of sample, and (c) determining a clearance normalized amount for the metabolite disease biomarker by normalizing the amount determined for the metabolite disease biomarker in step (a) to the amount of the kidney function biomarker determined in step (b). Moreover, the invention also relates to a method for diagnosing a disease in a subject suspected to suffer therefrom and to a device for determining a clearance normalized amount of a metabolite disease biomarker in a sample.

Small molecules such as various metabolites are usually excreted via the kidney. Accordingly, a proper kidney function it is decisive for a proper metabolite homeostasis. If the kidney function and, in particular, the glomerular filtration is impaired, the metabolites can no longer be excreted in usual amounts and may accumulate in the blood and other body fluids. Accordingly, the actual level for a given metabolite may be increased by improper kidney function rather than by other metabolic causes.

In the recent years, metabolic profiling has established a variety of promising metabolite disease markers for various diseases such as cardiovascular disease, metabolic diseases such as diabetes or metabolic syndrome or neurodegenerative disorders. It is essential to diagnose such disease efficiently and reliably. Usually, a disease causes an increase or a decrease, i.e. an alteration of the quantity, of metabolite biomarkers in body fluids such as blood. Such an increase or decrease will than be used as a diagnostic indicator for the presence, absence or risk for developing the disease. However, as set forth above, an improper kidney function may also affect the level of biomarkers in the blood including those which are suitable as disease metabolite biomarkers. Accordingly, a patient may be diagnosed to suffer from a disease such as a cardiovascular disease based on, e.g., an increase of a biomarker although said increase of the biomarker is caused by an impaired kidney function rather than by the cardiovascular disease. Accordingly, the patient will be classified falsely positive for the disease. Moreover, rather than addressing the renal impairment therapeutically, the patient will be treated for a probably non-existing cardiovascular disease.

Kidney function can be assessed by determining the glomerular filtration rate (GFR). To this end, creatinine clearance is conventionally determined. In addition to creatinine, GFR may also be determined by measuring the clearance of other compounds including exogenously applied once, such as inulins, or endogenous compounds, such as cystatin c (see, e.g., Grubb 1985, Acta Med Scand 218 (5): 499-503 or Simonsen 1985, Scand J Clin Invest 45(2): 97-101, Jernberg et al. (2004), Circulation 110:2342).

An impaired kidney function is normally taken into account for establishing a diagnosis by a medical practitioner. Although it was proposed by Badgwell et al. ((2007), Gynecologic Oncology 106(3):490) to normalize concentrations of cancer markers using the GFR determined by measuring serum and urine creatine, the kidney function parameter measured in blood or a derivative thereof has not been used to directly adjust or correct biomarker levels.

Nevertheless, it would be highly desirable to have comparable levels for biomarkers between all types of patients including those with impaired kidney function.

The technical problem underlying the present invention can be seen as the provision of means and methods for complying with the aforementioned needs. The said technical problem is solved by the embodiments characterized in the claims and herein below.

Thus, the present invention relates to a method for determining a clearance normalized amount of a metabolite disease biomarker in one type of sample comprising the steps of:
(a) determining the amount of the disease biomarker in at least a first type of sample of a subject suspected to suffer from the disease;
(b) determining the amount of a kidney function biomarker which correlates with the glomerular filtration rate (GFR) in the same type of sample; and
(c) determining a clearance normalized amount for the metabolite disease biomarker by normalizing the amount determined for the metabolite disease biomarker in step (a) to the amount of the kidney function biomarker determined in step (b),
wherein said type of sample is blood or a derivative thereof.

The method as referred to in accordance with the present invention includes a method which essentially consists of the aforementioned steps or a method which includes further steps. However, it is to be understood that the method, in a preferred embodiment, is a method carried out ex vivo, i.e. not practised on the human or animal body. The method, preferably, can be assisted by automation.

The term "clearance normalized amount" as used herein refers to an amount for a metabolite biomarker which is adjusted or corrected for kidney function and, in particular for renal clearance.

The term "sample" as disclosed herein encompasses any kind of biological sample which comprises metabolites and, preferably, also proteins. Accordingly, a sample as disclosed herein may be a biological fluid or a tissue or cell comprising sample. Preferably, the metabolites present in the said sample are affected by kidney function, i.e. their presence, absence and/or quantity may be altered by an impaired renal clearance. Typically samples such as blood or urine are immediately affected by the kidney function since improper renal clearance will, e.g., prevent the transmission of a metabolite from the blood into the urine. However, it will be understood that other samples such as saliva, liquor and the like may also secondarily be affected by an improper kidney function. According to the present invention, the sample is blood or a derivative thereof, such as plasma or serum or any other fraction of blood, or urine.

A first type of sample as referred to herein refers to either one first sample or different first samples from the same subject wherein said subject exhibits the same disease conditions when the said different first samples where taken and wherein the said different samples have been treated in an identical manner prior to the investigation by the method disclosed.

Accordingly, a second or further type of sample is to be understood as either one second or further sample or different second or further samples from a further subject being different from the subject from which the first type sample has been derived. Moreover, a second type of sample can also be derived from the subject from which the first type sample has been derived wherein said subject has underwent a treatment between deriving the first type sample and the second type sample or wherein a certain time period has passed between deriving the first type sample and the second type sample.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, fractioning, ultrafiltration, protein precipitation followed by filtration and purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

The term "subject" as used herein relates to animals and, preferably, to mammals. More preferably, the subject is a primate and, most preferably, a human. The subject, preferably, is suspected to suffer from a disease as specified herein.

The term "biomarker" as used herein refers to a molecular species which serves as an indicator for a disease or effect as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition. Moreover, a biomarker according to the present invention is not necessarily corresponding to one molecular species. Rather, the biomarker may comprise stereoisomers or enantiomeres of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters and, thus, in the case of, e.g., lipids an identical chain length and identical numbers of double bonds in the fatty acid and/or sphingo base moieties.

The term "kidney function biomarker" relates to a biomarker which is an indicator for proper kidney function, and, in particular, an indicator for proper renal clearance. Preferably, it is envisaged that such a kidney function biomarker is excreted under physiological conditions and, thus, present in a defined amount in, e.g., urine and/or blood. If the kidney function is impaired such that the renal clearance is altered for the said biomarker, the amounts present in either the urine or the blood or both will be changed, said change being indicative for the improper renal clearance and kidney function. Renal clearance is correlated to the glomerular filtration rate (GFR) which is the total amount of primary urine which is excreted by the glomeruli of the kidney within a certain period of time. The GFR is an essential parameter of proper kidney function. In human adults, the GFR is about 170 liters per day. Biomarkers which correlate to the GFR and which are, thus, suitable as kidney function biomarker in the sense of the instant invention encompass endogenous biomarker molecules, such as creatinine or cystatin c, or exogenously applied biomarker molecules, such as inulins.

Preferably, said kidney function biomarker is selected from the group consisting of cystatin C and creatinine. Cystatin c is a non-glycosylated, basic protein having an isoelectric point at about pH 9.3. Its three-dimensional structure is characterized by a short alpha helix and a long alpha helix running across a large antiparallel, five-stranded beta sheet. Cystatin c forms two disulfide bonds. About 50% of the cystatin c molecules in a subject carry a hydroxylated proline. Cystatine c forms dimers via subdomains wherein in the dimerized state, each half is made up of the long alpha helix and one beta strand of one partner and four beta strands of the other partner (see Janowski 2001, Nat Struct Biol 8(4): 316-320). Creatinine is a well known metabolite which results from creatine phosphate metabolic conversions in the muscle. More preferably, said kidney function biomarker is cystatin c.

The term "metabolite disease biomarker" as used herein refers to a biomarker which is an indicator for the presence of the disease or a predisposition for the disease. It will be, therefore, understood that the presence, absence or quantity of a metabolite disease biomarker as used herein is altered if a subject suffers from the disease or has a predisposition therefor. A disease in the sense of the present invention may be any health abnormality or disorder. Preferably, said metabolite disease biomarker is a biomarker for cardiovascular diseases or disorders, diabetes or metabolic syndrome or neurodegenerative diseases. Preferably, the said disease is a disease as recited in any one of tables 1 to 6 and 8 to 21. More preferably, said disease biomarker is a biomarker selected from any of tables 1 to 6 and 8 to 21. Moreover, it will be understood that more than one biomarker and up to all of the biomarkers recited in any one of the aforementioned tables may be determined as disease metabolite biomarker in the method of the present invention.

The term "determining the amount" as used herein refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

As discussed before, each biomarker comprised by a sample may be, preferably, determined in accordance with the present invention quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case were the precise amount of a biomarker can or shall not be determined. In said case, it can be determined whether the amount in which the biomarker is present is enlarged or diminished with respect to a second sample comprising said biomarker in a second amount. In a preferred embodiment said second sample comprising said biomarker shall be a calculated reference as specified elsewhere herein. Quantitatively analysing a biomarker, thus, also includes what is sometimes referred to as semi-quantitative analysis of a biomarker.

Moreover, determining as used in the method of the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be easily applied by the person skilled in the art without further ado. Most preferably, LC and/or GC are chromatographic techniques to be envisaged by the method of the present invention. Suitable devices for such determination of biomarkers are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Nissen 1995, Journal of Chromatography A, 703: 37-57, US 4,540,884 or US 5,397,894. As an alternative or in addition to mass spectrometry techniques, the following techniques may be used for compound determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.
Moreover, the at least one biomarker can also be determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes. Specific antibodies, for instance, may be obtained using the biomarker as antigen by methods well known in the art. Antibodies as referred to herein include both polyclonal and monoclonal antibodies, as well as fragments thereof, such as Fv, Fab and F(ab)2 fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the biomarker are, preferably, enzymes which are involved in the metabolic conversion of the said biomarker. Said enzymes may either use the biomarker as a substrate or may convert a substrate into the biomarker. Moreover, said antibodies may be used as a basis to generate oligopeptides which specifically recognize the biomarker. These oligopeptides shall, for example, comprise the enzyme's binding domains or pockets for the said biomarker. Suitable antibody and/or enzyme based assays may be RIA (radioimmunoassay), ELISA (enzymelinked immunosorbent assay), sandwich enzyme immune tests, electrochemiluminescence sandwich immunoassays (ECLIA), dissociation-enhanced lanthanide fluoroimmunoassay (DEL-FIA) or solid phase immune tests. Moreover, the biomarker may also be determined based on its capability to react with other compounds, i.e. by a specific chemical reaction. Further, the biomarker may be determined in a sample due to its capability to elicit a response in a biological read out system. The biological response shall be detected as read out indicating the presence and/or the amount of the biomarker comprised by the sample. The biological response may be, e.g., the induction of gene expression or a phenotypic response of a cell or an organism. In a preferred embodiment the determination of the least one biomarker is a quantitative process, e.g., allowing also the determination of the amount of the at least one biomarker in the sample.

As described above, said determining of the at least one biomarker can, preferably, comprise mass spectrometry (MS). Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO 03/073464.

More preferably, said mass spectrometry is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

The determination of the metabolite disease biomarker and the kidney function biomarker, preferably, may be carried out in the same aliquot of a type of sample or in different aliquots of a type of sample or aliquots of different samples of the same type. For example, the disease biomarker may be determined in an aliquot of a first first type sample and the kidney function biomarker is determined in an aliquot of a second first type sample. Alternatively, the disease biomarker may be determined in a first aliquot of a first sample and the kidney function biomarker may be determined in a second aliquot of said first sample. Still alternatively, the said disease biomarker and the kidney function biomarker may be determined simultaneously or consecutively in the same aliquot of a first sample.

A clearance normalized amount for the metabolite disease biomarker can be determined by any mathematical operation which establishes a relation between the determined amount of the metabolite disease biomarker and the amount of the kidney function biomarker such that the amount of the disease biomarker is put into relation to the kidney function. Such a relation can be established by adjusting or correcting the amount of the metabolite disease biomarker itself or by adjusting or correcting a parameter to be compared to the said amount of the metabolite disease biomarker such as a reference amount or threshold value. Preferably, the said normalizing in this context, i.e. in step (c) of the method of the invention, encompasses calculating a ratio of the amount determined for the disease biomarker in step (a) and the amount of the kidney function biomarker determined in step (b). It is to be understood that any form of correction for clearance effects can be carried out in accordance with the present invention in order to reflect a clearance normalized amount of a disease metabolite biomarker. For example, ratios, cut-off values or a linear and non-linear fits can be made. Moreover, also preferably encompassed is an analysis of variance (ANOVA) correction of the amount. By using ANOVA of parameters such as the amount of the metabolite disease biomarker in a diseased and a healthy group of subjects and the amount of a kidney function biomarker such as cystatin C, a correction factor can be calculated reflecting the difference between the predicted fit and the actual test fit underlying the ANOVA. Said correction factor can subsequently be applied to normalize an amount of the metabolite disease biomarker. Preferably, ANOVA correction is used for reflecting clearance normalized amounts for at least one metabolite disease biomarker in a comparison of different study set ups, such as different cohorts of subjects to be compared with each other with respect to the amount of at least one metabolite disease biomarker. Further, clearance normalization according to the invention may be, preferably, achieved by adjusting or correcting a reference amount or threshold value.

In a preferred embodiment of the method of the present invention, steps (a) and (b) are carried out for a second type of sample being different from the first type of sample and wherein said normalizing in step (c) encompasses calculating (i) a ratio of the amount determined for the metabolite disease biomarker in the first type and the second type samples, (ii) calculating a ratio of the kidney function biomarker determined in the first type and the second type samples, and (iii) calculating a ratio of the ratios calculated under (i) and (ii). Also preferably, said normalizing can encompass (i) determining the ratio of the metabolite biomarker and the kidney biomarker in the first sample and (ii) the ratio of the metabolite biomarker and the kidney biomarker in the second sample.

Advantageously, it has been found in accordance with the present invention that an impaired kidney function including improper renal clearance affects the blood metabolite levels of metabolic biomarkers. In particular, levels for metabolites in the blood including those which can serve as biomarkers are, in general, increased due to an improper removal by renal excretion. As a consequence, patients suffering from impaired kidney function may be diagnosed to suffer from disease base on said increased levels of metabolic biomarkers. However, in such patients, the change in the biomarker level is not caused by a disease but rather by an improper kidney function. Thanks to the present invention, a clearance normalized amount for a disease metabolite biomarker can be determined by taking into account a kidney function biomarker as a normalization parameter for the determined amount of one or several disease biomarker. Moreover, thanks to the said normalization, amounts for disease biomarkers can be compared between individuals more reliably since inter-individual clearance differences will be efficiently reduced. Thus, threshold amount such as the upper limits for physiological amounts for a biomarker can be more reliably established. More specifically, it was found in the studies underlying the present invention that a plurality of plasma metabolites correlated with cystatin c levels and, thus, with kidney function. Moreover, the data quality for plasma metabolites could be significantly improved after normalization to the cystatin c levels.

The definitions and explanations of the terms made above apply mutatis mutandis for the following embodiments of the present invention except specified otherwise herein below.

The present invention also relates to a method for diagnosing a disease in a subject suspected to suffer therefrom comprising:
(a) determining a clearance normalized amount for at least one metabolite disease biomarker in a sample of said subject according to the method of the invention specified above; and
(b) comparing said clearance normalized amount to a reference, whereby the disease is to be diagnosed.

The term "diagnosing" as used herein refers to assessing whether a subject suffers from a disease as specified herein, or not. As will be understood by those skilled in the art, such an assessment, although preferred to be, may usually not be correct for 100% of the investigated subjects. The term, however, requires that a statistically significant portion of subjects can be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.2, 0.1, or 0.05.

The term includes individual diagnosis of a disease or its symptoms as well as continuous monitoring of a patient. Monitoring, i.e. diagnosing the presence or absence of the disease or the symptoms accompanying it at various time points, includes monitoring of patients known to suffer from the said disease as well as monitoring of subjects known to be at risk of developing the disease. Furthermore, monitoring can also be used to determine whether a patient is treated successfully or whether at least symptoms of the disease can be ameliorated over time by a certain therapy.

The term "disease" as used herein refers to any health abnormality or disorder in a subject. Preferably, said disease is a cardiovascular disease or disorder and, more preferably, congestive heart failure, diabetes or metabolic syndrome and, more preferably, diabetes type II, or neurodegenerative diseases and, more preferably, multiple sclerosis. More preferably, the said disease is a disease as recited in any one of tables 1 to 6 and 8 to 21.

The term "reference" refers to values of characteristic features of each of the biomarker which can be correlated to a medical condition, i.e. the presence or absence of the disease referred to herein. Preferably, a reference is a threshold value (e.g., an amount or ratio of amounts) for a biomarker whereby values found in a sample to be investigated which are higher than or essentially identical to the threshold are indicative for the presence of a medical condition while those being lower are indicative for the absence of the medical condition. It will be understood that also preferably, a reference may be a threshold value for a biomarker whereby values found in a sample to be investigated which are lower or identical than the threshold are indicative for the presence of a medical condition while those being higher are indicative for the absence of the medical condition.

In accordance with the aforementioned method of the present invention, a reference is, preferably, a reference obtained from a sample from a subject or group of subjects known to suffer from a disease as referred to herein. In such a case, a value for the at least one biomarker found in the test sample being essentially identical is indicative for the presence of the disease.

Moreover, the reference, also preferably, could be from a subject or group of subjects known not to suffer from a disease as referred to herein, preferably, an apparently healthy subject or group of healthy subjects. In such a case, a value for the at least one biomarker found in the test sample being altered with respect to the reference is indicative for the presence of the disease. The same applies mutatis mutandis for a calculated reference being, most preferably, the average or median for the relative value or the value for a degree of change of the at least one biomarker in a population of individuals (comprising the subject to be investigated). The relative values or degrees of changes of the at least one biomarker of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

The value for the at least one biomarker of the test sample and the reference values are essentially identical, if the values for the characteristic features and, in the case of quantitative determination, the intensity values are essentially identical. Essentially identical means that the difference between two values is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value, preferably, the 50th, 60th, 70th, 80th, 90th or 95th percentile of the reference value. Statistical test for determining whether two amounts are essentially identical are well known in the art and are also described elsewhere herein.

An observed difference for two values, on the other hand, shall be statistically significant. A difference in the relative or absolute value is, preferably, significant outside of the interval between 45th and 55th percentile, 40th and 60th percentile, 30th and 70th percentile, 20th and 80th percentile, 10th and 90th percentile, 5th and 95th percentile, 1st and 99th percentile of the reference value. Preferred relative changes of the medians or degrees of changes are described in the accompanying Tables as well as in the Examples. In the Tables below, a preferred relative change for the biomarkers is indicated as "up" for an increase and "down" for a decrease in column "direction of change". Values for preferred degrees of changes are indicated in the column "estimated fold change". The preferred references for the aforementioned relative changes or degrees of changes are indicated in the Tables below as well. It will be understood that these changes are, preferably, observed in comparison to the references indicated in the respective Tables, below.

Preferably, the reference, i.e. values for at least one characteristic feature of the at least one biomarker or ratios thereof, will be stored in a suitable data storage medium such as a database and are, thus, also available for future assessments.

The term "comparing" refers to determining whether the determined value of a biomarker is essentially identical to a reference or differs there from. Preferably, a value for a biomarker is deemed to differ from a reference if the observed difference is statistically significant which can be determined by statistical techniques referred to elsewhere in this description. If the difference is not statistically significant, the biomarker value and the reference are essentially identical. Based on the comparison referred to above, a subject can be assessed to suffer from the disease, or not.

For the specific biomarkers referred to in this specification, preferred values for the changes in the relative amounts or ratios (i.e. the changes expressed as the ratios of the medians) are found in the Tables, below.

The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

Moreover, the present invention relates to the use of a kidney function biomarker as defined elsewhere herein in a sample of a subject comprising a metabolite disease biomarker for normalizing said metabolite disease biomarker, wherein said sample is blood or a derivative thereof.

Further the present disclosure relates to the use of a kidney function biomarker as defined elsewhere herein for manufacturing a diagnostic pharmaceutical composition for normalizing in a sample of a subject comprising a metabolite disease biomarker said metabolite disease biomarker.

The invention further relates to a device for determining a clearance normalized amount of a metabolite disease biomarker in a sample comprising:
a) an analyzing unit comprising a detection agent which specifically detects the amount of at least one metabolite disease biomarker in at least a first type of sample and a detection agent which specifically detects the amount of a kidney function biomarker in said at least first type of sample; and
b) an evaluation unit comprising a data processor having tangibly embedded a computer program code carrying out an algorithm which normalizes the amount for the metabolite disease biomarker to the amount of the kidney function biomarker,
wherein said normalization encompasses calculating a ratio of the amount determined for the metabolite disease biomarker and the amount of the kidney function biomarker.

A device as used herein shall comprise at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Said device may accordingly include an analyzing unit for the biomarker and for the kidney function biomarker and a computer or data processing device as evaluation unit for processing the resulting data for the assessment and for stabling the output information. Preferred devices are those which can be applied without the particular knowledge of a specialized clinician, e.g., electronic devices which merely require loading with a sample. The output information of the device, preferably, is a numerical value for the clearance normalized amount of a metabolite disease biomarker.

In a preferred embodiment of the device of the invention, said evaluation unit comprises a database with stored references which allow for diagnosing a disease based on the clearance normalized amount for the metabolite disease biomarker. In this case, the output information of the device allows drawing conclusions on the presence or absence of a disease and, thus, is an aid for diagnosis. More preferably, the output information is a preliminary diagnosis or an aid for diagnosis based on the aforementioned numerical value, i.e. a classifier which indicates whether the subject suffers from a disease or not. Such a preliminary diagnosis may need the evaluation of further information which can be provided in the device of the invention by including an expert knowledge database system.

A preferred reference to be used as a stored reference in accordance with the device of the present invention is an amount for the at least one biomarker to be analyzed or values derived therefrom which are derived from a subject or group of subjects known to suffer from a disease. More preferably the stored reference in accordance with the device of the present invention is an clearance normalized amount for the at least one biomarker to be analysed. In such a case, the algorithm tangibly embedded, preferably, compares the determined amount for the at least one clearance normalised biomarker with the clearance normalised reference wherein an identical or essentially identical amount or value shall be indicative for the presence of the disease in the subject.

Alternatively, another preferred reference to be used as a stored reference in accordance with the device of the present invention is an amount for the at least one biomarker to be analyzed or values derived therefrom which are derived from a subject or group of subjects known not to suffer from a disease. In such a case, the algorithm tangibly embedded, preferably, compares the determined amount for the at least one biomarker with the reference wherein an amount or value which differs from the reference shall be indicative for the presence of the disease in the subject. Preferred differences are those indicated as relative changes or degrees of changes for the individual biomarkers in the Tables below.

The units of the device, also preferably, can be implemented into a system comprising several devices which are operatively linked to each other. Depending on the units to be used for the system of the present invention, said means may be functionally linked by connecting each mean with the other by means which allow data transport in between said means, e.g., glass fiber cables, and other cables for high throughput data transport. Nevertheless, wireless data transfer between the means is also envisaged by the present invention, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS are used in the system of the present invention as described in detail elsewhere in the specification. Further comprised shall be means for comparing and/or analyzing the results obtained from the means for determination of biomarkers. The means for comparing and/or analyzing the results may comprise at least one databases and an implemented computer program for comparison of the results. Preferred embodiments of the aforementioned systems and devices are also described in detail below.

As set forth elsewhere herein, the normalization carried out by the evaluation unit encompasses an algorithm for calculating a ratio of the amount determined for the metabolite disease biomarker in step (a) and the amount of the kidney function biomarker determined in step (b). Said algorithm can be implemented by a computer program code tangibly embedded in a data processor comprised in the evaluation unit.

The invention will now be illustrated by the following Examples which are not intended to restrict or limit the scope of this invention.

### EXAMPLES

### Example 1: Disease associated metabolite biomarkers

In the following tables 1 to 6, 8, and 9 to 21 disease related metabolite biomarkers are listed. The biomarkers can be determined and analyzed as described in any one of WO2011/092285 A2, WO2012/085890, WO2007/110357 or WO2007/110358. The nomenclature of lipids from the analysis of complex lipids has been applied like described in WO2011/092285.

Cardiac markers according to WO2011/092285:
Biomarkers not precisely defined by their name in any one of tables 1 to 6 are further characterized in table 7.

**Table 1a: Metabolites with a significant difference (p-value < 0.05) between patients with congestive heart failure (CHF) and healthy controls**

| Metabolite_Name | ratio of median | regulation | p-value |
|---|---|---|---|
| Lysophosphatidylcholine (C18:2) | 0.656 | down | 0.000002 |
| Mannose | 1.949 | up | 0.000000 |
| Hypoxanthine | 2.136 | up | 0.000006 |
| Phytosphingosine | 0.779 | down | 0.000010 |
| Lignoceric acid (C24:0) | 0.654 | down | 0.000029 |
| Glutamate | 2.027 | up | 0.000037 |
| 2-Hydroxybutyrate | 1.724 | up | 0.000132 |
| Lysophosphatidylcholine (C18:0) | 0.820 | down | 0.000213 |
| Behenic acid (C22:0) | 0.744 | down | 0.000224 |
| Tricosanoic acid (C23:0) | 0.708 | down | 0.000237 |
| Phosphatidylcholine (C18:0, C18:2) | 1.028 | up | 0.000248 |
| Linoleic acid (C18:cis[9,12]2) | 0.733 | down | 0.000270 |
| Pseudouridine | 1.299 | up | 0.000321 |
| Phosphate, lipid fraction | 0.817 | down | 0.000333 |
| Lysophosphatidylcholine (C18:1) | 0.874 | down | 0.000432 |
| Lysophosphatidylcholine (C17:0) | 0.770 | down | 0.000612 |
| erythro-Sphingosine (*1) | 0.823 | down | 0.000620 |
| Glycerol phosphate, lipid fraction | 0.768 | down | 0.000628 |
| 5-O-Methylsphingosine (*1) | 0.802 | down | 0.000766 |
| Galactose, lipid fraction | 0.775 | down | 0.000846 |
| Cholesterol | 0.855 | down | 0.000921 |
| alpha-Ketoglutarate | 1.235 | up | 0.000944 |
| Histidine | 0.790 | down | 0.000945 |
| Eicosanoic acid (C20:0) | 0.835 | down | 0.001148 |
| 3-O-Methylsphingosine (*1) | 0.769 | down | 0.001248 |
| erythro-C16-Sphingosine | 0.827 | down | 0.001492 |
| Uric acid | 1.429 | up | 0.001696 |
| Cholestenol No 02 | 0.821 | down | 0.004244 |
| Urea | 1.243 | up | 0.005073 |
| Adrenaline (Epinephrine) | 1.926 | up | 0.006118 |
| Aspartate | 1.120 | up | 0.006265 |
| Normetanephrine | 1.262 | up | 0.006469 |
| Pentadecanol | 0.583 | down | 0.006875 |
| myo-Inositol, lipid fraction | 0.775 | down | 0.007379 |
| Dehydroepiandrosterone sulfate | 0.594 | down | 0.007754 |
| Phosphatidylcholine (C16:1, C18:2) | 0.883 | down | 0.008776 |
| Sphingomyelin (d18:1, C24:0) | 0.943 | down | 0.011533 |
| Threonine | 0.855 | down | 0.012287 |
| myo-Inositol-2-phosphate, lipid fraction (myo-Inositolphospholipids) | 0.635 | down | 0.012637 |
| Myristic acid (C14:0) | 0.572 | down | 0.015030 |
| Homovanillic acid (HVA) | 1.292 | up | 0.015937 |
| Arginine | 0.844 | down | 0.016192 |
| Glutamine | 0.850 | down | 0.016336 |
| Elaidic acid (C18:trans[9]1) | 1.267 | up | 0.017410 |
| 4-Hydroxy-3-methoxyphenylglycol (HMPG) | 1.128 | up | 0.019069 |
| Cystine | 1.105 | up | 0.020208 |
| 4-Hydroxy-3-methoxymandelic acid | 1.179 | up | 0.020480 |
| Zeaxanthin | 0.699 | down | 0.021888 |
| Glucose | 1.215 | up | 0.023219 |
| Stearic acid (C18:0) | 0.918 | down | 0.023703 |
| Cortisol | 1.345 | up | 0.025615 |
| 3-Methoxytyrosine | 1.209 | up | 0.026958 |
| 5-Hydroxy-3-indoleacetic acid (5-HIAA) | 1.255 | up | 0.027467 |
| Lysophosphatidylcholine (C20:4) | 0.944 | down | 0.029167 |
| Creatinine | 1.208 | up | 0.031253 |
| Heptadecanoic acid (C17:0) | 0.828 | down | 0.032349 |
| Proline | 0.818 | down | 0.033617 |
| Erythrol | 1.224 | up | 0.035087 |
| Nervonic acid (C24:cis[15]1) | 0.879 | down | 0.035240 |
| Coenzyme Q10 | 1.060 | up | 0.036613 |
| Coenzyme Q9 | 0.774 | down | 0.040228 |
| Phosphatidylcholine (C18:0, C18:1) | 0.966 | down | 0.044253 |
| Cryptoxanthin | 0.464 | down | 0.047617 |
| 1,5-Anhydrosorbitol | 0.808 | down | 0.047807 |
| SM_Sphingomyelin (d17:1,C24:0) | 0.7142 | down | 2.8E-13 |
| SM_Sphingomyelin (d17:1,C22:0) | 0.7423 | down | 9.8E-12 |
| SM_Sphingomyelin (d17:1,C23:0) | 0.6392 | down | 1.4E-11 |
| CE_Cholesterylester C15:0 | 0.6745 | down | 8.8E-11 |
| Cholesterylester C18:2 | 0.7013 | down | 2.1E-10 |
| SM_Sphingomyelin (d16:1,C23:0) | 0.7103 | down | 2.7E-10 |
| Isocitrate | 1.2983 | up | 4.6E-10 |
| 1-Hydroxy-2-amino-(cis,trans)-3,5-octadecadiene (from sphingolipids) | 0.738 | down | 1.2E-09 |
| Noradrenaline (Norepinephrine) | 1.5067 | up | 4.9E-09 |
| SM_Sphingomyelin (d16:1,C22:0) | 0.7499 | down | 8.7E-09 |
| SM_Sphingomyelin (d16:1,C24:0) | 0.6773 | down | 1.1E-08 |
| Maltose | 1.8136 | up | 1.9E-08 |
| SM_Sphingomyelin (d18:2,C23:0) | 0.8134 | down | 2.7E-08 |
| SM_Sphingomyelin (d17:1,C20:0) | 0.7884 | down | 3E-08 |
| SM_Sphingomyelin (d17:1,C16:0) | 0.8169 | down | 1.6E-07 |
| SM_Sphingomyelin (d18:1,C14:0) | 0.8274 | down | 2.5E-07 |
| CE_Cholesterylester C14:0 | 0.7641 | down | 5.2E-07 |
| Sphingomyelin (d18:1,C23:0) | 0.8793 | down | 6.2E-07 |
| CER_Ceramide (d17:1,C24:0) | 0.7452 | down | 1.7E-06 |
| SM_Sphingomyelin (d18:2,C24:0) | 0.834 | down | 2.3E-06 |
| Uridine | 0.7617 | down | 3.4E-06 |
| CER_Ceramide (d18:2,C14:0) | 0.7732 | down | 6.9E-06 |
| CER_Ceramide (d17:1,C23:0) | 0.7443 | down | 9E-06 |
| SM_Sphingomyelin (d16:1,C20:0) | 0.8091 | down | 1E-05 |
| SM_Sphingomyelin (d17:1,C24:1) | 0.8482 | down | 2.2E-05 |
| SM_Sphingomyelin (d17:1,C18:0) | 0.8393 | down | 3E-05 |
| CE_Cholesterylester C22:6 | 0.7561 | down | 3.3E-05 |
| SM_Sphingomyelin (d16:1,C22:1) | 0.8034 | down | 3.6E-05 |
| myo-Inositol | 1.16 | up | 4.6E-05 |
| CER_Ceramide (d16:1,C24:0) | 0.762 | down | 6.7E-05 |
| beta-Carotene | 0.7066 | down | 8.1E-05 |
| SM_Sphingomyelin (d16:1,C24:1) | 0.8446 | down | 0.00011 |
| Ornithine | 1.1516 | up | 0.00012 |
| SM_Sphingomyelin (d18:2,C22:0) | 0.8501 | down | 0.00013 |
| Cholesta-2,4,6-triene | 0.8494 | down | 0.00016 |
| TAG (C16:0,C18:2) | 1.3317 | up | 0.00017 |
| CE_Cholesterylester C16:2 | 0.7746 | down | 0.00017 |
| CE_Cholesterylester C20:5 | 0.7085 | down | 0.00018 |
| Sorbitol | 1.5523 | up | 0.00019 |
| SM_Sphingomyelin (d18:2,C23:1) | 0.8561 | down | 0.00021 |
| Isopalmitic acid (C16:0) | 0.7684 | down | 0.00022 |
| Sarcosine | 1.1039 | up | 0.00024 |
| Phosphatidylcholine (C18:2,C20:4) | 0.9367 | down | 0.00025 |
| CER_Ceramide (d18:1,C14:0) | 0.8316 | down | 0.00026 |
| SM_Sphingomyelin (d16:1,C18:1) | 0.8335 | down | 0.00031 |
| Sphingosine-1-phosphate (d17:1) | 0.8268 | down | 0.00032 |
| TAG (C16:0,C18:1,C18:2) | 1.4134 | up | 0.00034 |
| SM_Sphingomyelin (d16:1,C21:0) | 0.8077 | down | 0.00038 |
| CER_Ceramide (d16:1,C23:0) | 0.7763 | down | 0.00038 |
| Docosahexaenoic acid (C22:cis[4,7,10,13,16,19]6) | 0.7778 | down | 0.00044 |
| TAG (C18:1 ,C18:2) | 1.3426 | up | 0.00053 |
| Tyrosine | 1.1292 | up | 0.00057 |
| Testosterone | 0.7956 | down | 0.00059 |
| threo-Sphingosine (*1) | 0.8766 | down | 0.00078 |
| Phenylalanine | 1.0929 | up | 0.00081 |
| CE_Cholesterylester C14:1 | 0.68 | down | 0.00082 |
| Cholesta-2,4-dien | 0.8533 | down | 0.00096 |
| SM_Sphingomyelin (d16:1,C16:0) | 0.8766 | down | 0.00114 |
| Malate | 1.1907 | up | 0.00116 |
| SM_Sphingomyelin (d18:1,C22:0) | 0.8379 | down | 0.00119 |
| CE_Cholesterylester C16:3 | 0.7918 | down | 0.00122 |
| 5-Oxoproline | 1.0814 | up | 0.00123 |
| CE_Cholesterylester C22:5 | 0.8603 | down | 0.00125 |
| SM_Sphingomyelin (d18:1,C23:1) | 0.8878 | down | 0.00132 |
| Docosapentaenoic acid (C22:cis[7,10,13,16,19]5) | 0.8085 | down | 0.00165 |
| CER_Ceramide (d17:1,C16:0) | 0.8577 | down | 0.00176 |
| Taurine | 1.1928 | up | 0.00178 |
| Phosphatidylcholine (C16:0,C20:5) | 0.9159 | down | 0.00195 |
| SM_Sphingomyelin (d18:2,C14:0) | 0.871 | down | 0.00207 |
| Cholesterylester C18:1 | 0.8256 | down | 0.00219 |
| CER_Ceramide (d17:1,C22:0) | 0.8324 | down | 0.00247 |
| CE_Cholesterylester C18:3 | 0.7933 | down | 0.00311 |
| CER_Ceramide (d18:1,C18:0) | 1.1562 | up | 0.00456 |
| SM_Sphingomyelin (d18:2,C21:0) | 0.8893 | down | 0.00466 |
| CE_Cholesterylester C18:4 | 0.7197 | down | 0.00569 |
| SM_Sphingomyelin (d16:1,C18:0) | 0.8762 | down | 0.0057 |
| Glycerol-3-phosphate, polar fraction | 1.159 | up | 0.00613 |
| Cholesterylester C16:0 | 0.8225 | down | 0.00685 |
| Eicosapentaenoic acid (C20:cis[5,8,11,14,17]5) | 0.7853 | down | 0.00809 |
| CE_Cholesterylester C12:0 | 0.7224 | down | 0.00887 |
| trans-4-Hydroxyproline | 1.2178 | up | 0.0089 |
| SM_Sphingomyelin (d18:1,C21:0) | 0.9157 | down | 0.00945 |
| CER_Ceramide (d18:2,C23:0) | 0.869 | down | 0.00948 |
| TAG (C16:0,C16:1) | 1.2811 | up | 0.01131 |
| Glycerol, lipid fraction | 1.2809 | up | 0.01216 |
| CER_Ceramide (d16:1,C16:0) | 0.8776 | down | 0.0122 |
| Cysteine | 1.0714 | up | 0.01409 |
| Phosphatidylcholine (C16:0,C20:4) | 0.991 | down | 0.01571 |
| 8-Hydroxyeicosatetraenoic acid (C20:trans[5]cis[9,11,14]4) (8-HETE) | 1.2207 | up | 0.01617 |
| Hippuric acid | 0.7043 | down | 0.01627 |
| Sphingosine (d18:1) | 1.264 | up | 0.01632 |
| SM_Sphingomyelin (d18:2,C18:1) | 0.9068 | down | 0.01633 |
| Hexadecanol | 1.1092 | up | 0.01765 |
| 14-Methylhexadecanoic acid | 0.8393 | down | 0.01844 |
| CER_Ceramide (d16:1,C22:0) | 0.8608 | down | 0.02052 |
| CER_Ceramide (d18:2,C24:0) | 0.8903 | down | 0.02079 |
| SM_Sphingomyelin (d18:2,C24:2) | 0.9157 | down | 0.02116 |
| Creatine | 1.1628 | up | 0.02211 |
| Eicosenoic acid (C20:cis[11]1) | 1.1674 | up | 0.02337 |
| 14,15-Dihydroxyeicosatrienoic acid (C20:cis[5,8,11]3) | 1.1603 | up | 0.0238 |
| Sphinganine (d18:0) | 1.2016 | up | 0.02412 |
| CER_Ceramide (d18:1,C23:0) | 0.8973 | down | 0.02646 |
| CER_Ceramide (d17:1,C20:0) | 0.876 | down | 0.02705 |
| CER_Ceramide (d18:1,C24:0) | 0.8982 | down | 0.02746 |
| Fumarate | 1.051 | up | 0.03023 |
| SM_Sphingomyelin (d18:2,C20:0) | 0.9289 | down | 0.03273 |
| conjugated Linoleic acid (C18:trans[9,11]2) | 0.8624 | down | 0.03361 |
| 13-Hydroxyoctadecadienoic acid (13-HODE) (C18:cis[9]trans[11]2) | 1.1549 | up | 0.03371 |
| Campesterol | 0.8211 | down | 0.03589 |
| 3,4-Dihydroxyphenylalanine (DOPA) | 1.0983 | up | 0.03675 |
| TAG (C18:2,C18:2) | 1.2038 | up | 0.03696 |
| Phosphatidylcholine No 02 | 0.9467 | down | 0.03922 |
| Glucose-1-phosphate | 1.089 | up | 0.03978 |
| CER_Ceramide (d17:1,C24:1) | 0.8986 | down | 0.04172 |
| Lactaldehyde | 1.0876 | up | 0.04225 |
| Methionine | 1.0698 | up | 0.04311 |
| Lysophosphatidylethanolamine (C22:5) | 0.9229 | down | 0.04472 |
| scyllo-Inositol | 1.1685 | up | 0.04903 |
| CER_Ceramide (d16:1,C21:0) | 0.8656 | down | 0.04997 |

| | | | |
|---|---|---|---|
| (*1): free and from sphingolipids | | | |

**Table 1b: Metabolites of table 1a which additionally showed a significant difference (p-value < 0.1) between ischemic cardiomyopathy (ICMP) patients and healthy controls**

| Metabolite_Name | ratio of median | regulation | p-value |
|---|---|---|---|
| Cholesterylester C18:2 | 0,6066 | down | 3,17E-17 |
| SM_Sphingomyelin (d18:1,C14:0) | 0,7751 | down | 3,88E-11 |
| SM_Sphingomyelin (d18:2,C23:0) | 0,7837 | down | 3,14E-10 |
| SM_Sphingomyelin (d17:1,C23:0) | 0,661 | down | 1,21E-09 |
| Tricosanoic acid (C23:0) | 0,7527 | down | 2,78E-09 |
| CE_Cholesterylester C15:0 | 0,6948 | down | 5,44E-09 |
| SM_Sphingomyelin (d17:1,C24:0) | 0,7656 | down | 1,24E-08 |
| 1-Hydroxy-2-amino-(cis,trans)-3,5-octadecadiene (from sphingolipids) | 0,7463 | down | 1,33E-08 |
| Sorbitol | 1,9715 | up | 3,76E-08 |
| SM_Sphingomyelin (d17:1,C16:0) | 0,8059 | down | 6,53E-08 |
| SM_Sphingomyelin (d16:1,C23:0) | 0,7416 | down | 7,29E-08 |
| beta-Carotene | 0,6178 | down | 1,71E-07 |
| Glutamate | 1,4858 | up | 2,7E-07 |
| CE_Cholesterylester C14:0 | 0,7622 | down | 2,73E-07 |
| SM_Sphingomyelin (d18:2,C23:1) | 0,8017 | down | 4,36E-07 |
| Cholesterylester C18:1 | 0,7308 | down | 4,92E-07 |
| SM_Sphingomyelin (d18:2,C24:0) | 0,82 | down | 6,35E-07 |
| SM_Sphingomyelin (d17:1,C22:0) | 0,8018 | down | 6,9E-07 |
| SM_Sphingomyelin (d18:2,C24:2) | 0,82 | down | 7,56E-07 |
| Lignoceric acid (C24:0) | 0,7793 | down | 8,82E-07 |
| TAG (C16:0,C18:2) | 1,4494 | up | 9,3E-07 |
| threo-Sphingosine (*1) | 0,8271 | down | 1,11E-06 |
| SM_Sphingomyelin (d16:1,C24:0) | 0,7192 | down | 2,4E-06 |
| Sphingomyelin (d18:1,C23:0) | 0,8821 | down | 2,52E-06 |
| Phosphatidylcholine (C16:0,C20:4) | 0,9828 | down | 2,97E-06 |
| Lysophosphatidylcholine (C17:0) | 0,8091 | down | 3,34E-06 |
| Cholesterol, total | 0,8639 | down | 3,68E-06 |
| SP_Sphingosine-1-phosphate (d17:1) | 0,7871 | down | 4,86E-06 |
| TAG (C16:0,C18:1,C18:2) | 1,5361 | up | 7,11E-06 |
| Glucose | 1,1273 | up | 8,77E-06 |
| SM_Sphingomyelin (d17:1,C24:1) | 0,8464 | down | 1,25E-05 |
| TAG (C18:1 ,C18:2) | 1,439 | up | 1,53E-05 |
| Isocitrate | 1,2014 | up | 1,7E-05 |
| Phosphatidylcholine (C18:0,C18:2) | 1,0183 | up | 2,19E-05 |
| Zeaxanthin | 0,7372 | down | 2,46E-05 |
| CER_Ceramide (d18:1,C18:0) | 1,2527 | up | 2,54E-05 |
| Cysteine | 1,1313 | up | 2,62E-05 |
| SM_Sphingomyelin (d18:1 ,C23:1) | 0,8504 | down | 2,65E-05 |
| Behenic acid (C22:0) | 0,839 | down | 2,7E-05 |
| Maltose | 1,5712 | up | 2,99E-05 |
| Uric acid | 1,1916 | up | 2,99E-05 |
| erythro-C16-Sphingosine | 0,7823 | down | 3,62E-05 |
| SM_Sphingomyelin (d18:2,C14:0) | 0,8257 | down | 4,08E-05 |
| Cholesta-2,4-dien | 0,8257 | down | 5,49E-05 |
| Glucose-1-phosphate | 1,1806 | up | 5,61E-05 |
| 5-O-Methylsphingosine (*1) | 0,827 | down | 6,28E-05 |
| Glycerol, lipid fraction | 1,4758 | up | 7E-05 |
| Pseudouridine | 1,1483 | up | 7,79E-05 |
| TAG (C16:0,C16:1) | 1,4548 | up | 0,000109 |
| SM_Sphingomyelin (d18:2,C22:0) | 0,8469 | down | 0,00015 |
| Cholesta-2,4,6-triene | 0,8518 | down | 0,000167 |
| SM_Sphingomyelin (d16:1,C22:0) | 0,8256 | down | 0,00017 |
| SM_Sphingomyelin (d16:1 ,C24:1) | 0,845 | down | 0,000187 |
| erythro-Sphingosine (*1) | 0,8619 | down | 0,000211 |
| Cystine | 1,2256 | up | 0,00026 |
| Linoleic acid (C18:cis[9,12]2) | 0,8234 | down | 0,000276 |
| 3-O-Methylsphingosine (*1) | 0,839 | down | 0,000315 |
| Taurine | 1,2195 | up | 0,000362 |
| CER_Ceramide (d18:1,C14:0) | 0,8309 | down | 0,000397 |
| Dehydroepiandrosterone sulfate | 0,6197 | down | 0,000427 |
| Lysophosphatidylcholine (C18:2) | 0,8578 | down | 0,000485 |
| 14,15-Dihydroxyeicosatrienoic acid (C20:cis[5,8,11]3) | 1,2659 | up | 0,000573 |
| CER_Ceramide (d17:1,C23:0) | 0,7911 | down | 0,000631 |
| TAG (C18:2,C18:2) | 1,3485 | up | 0,000677 |
| SM_Sphingomyelin (d16:1,C16:0) | 0,8677 | down | 0,000709 |
| Erythrol | 1,1759 | up | 0,000711 |
| CE_Cholesterylester C12:0 | 0,6467 | down | 0,000734 |
| SM_Sphingomyelin (d16:1 ,C22:1) | 0,8327 | down | 0,000787 |
| Phytosphingosine, total | 0,8621 | down | 0,000895 |
| alpha-Ketoglutarate | 1,1818 | up | 0,000916 |
| 8-Hydroxyeicosatetraenoic acid (C20:trans[5]cis[9,11,14]4) (8-HETE) | 1,3254 | up | 0,001168 |
| CER_Ceramide (d17:1,C24:0) | 0,8152 | down | 0,001205 |
| Cholesterylester C16:0 | 0,788 | down | 0,00143 |
| CE_Cholesterylester C14:1 | 0,7029 | down | 0,001854 |
| SM_Sphingomyelin (d18:1,C22:0) | 0,8429 | down | 0,002434 |
| SM_Sphingomyelin (d18:2,C21:0) | 0,8781 | down | 0,002466 |
| Eicosenoic acid (C20:cis[11]1) | 1,2263 | up | 0,002476 |
| Sarcosine | 1,0878 | up | 0,002491 |
| Adrenaline (Epinephrine) | 1,4435 | up | 0,002549 |
| Galactose, lipid fraction | 0,8964 | down | 0,002702 |
| SM_Sphingomyelin (d17:1,C20:0) | 0,8783 | down | 0,002949 |
| Isoleucine | 1,1085 | up | 0,00385 |
| Isopalmitic acid (C16:0) | 0,8172 | down | 0,003877 |
| CER_Ceramide (d18:2,C14:0) | 0,8446 | down | 0,004044 |
| CE_Cholesterylester C16:2 | 0,8272 | down | 0,004416 |
| Normetanephrine | 1,2896 | up | 0,004728 |
| trans-4-Hydroxyproline | 1,2407 | up | 0,005701 |
| 4-Hydroxy-3-methoxymandelic acid | 1,6034 | up | 0,005745 |
| Mannose | 1,1511 | up | 0,006205 |
| CE_Cholesterylester C22:5 | 0,8782 | down | 0,006918 |
| 5-Oxoproline | 1,0658 | up | 0,007306 |
| myo-Inositol | 1,1023 | up | 0,009187 |
| CE_Cholesterylester C22:6 | 0,8366 | down | 0,009822 |
| SM_Sphingomyelin (d16:1,C21:0) | 0,8596 | down | 0,010056 |
| CER_Ceramide (d16:1,C23:0) | 0,8277 | down | 0,010099 |
| Lysophosphatidylcholine (C18:0) | 0,9017 | down | 0,011903 |
| Ornithine | 1,0943 | up | 0,012027 |
| Noradrenaline (Norepinephrine) | 1,194 | up | 0,01265 |
| SM_Sphingomyelin (d16:1,C18:1) | 0,8798 | down | 0,013795 |
| 3-Methoxytyrosine | 1,1696 | up | 0,016194 |
| Cholestenol No 02 | 0,9013 | down | 0,016563 |
| CE_Cholesterylester C18:3 | 0,8332 | down | 0,01764 |
| CER_Ceramide (d16:1,C24:0) | 0,8487 | down | 0,019382 |
| Sphingomyelin (d18:1,C24:0) | 0,9423 | down | 0,020541 |
| Testosterone | 0,8537 | down | 0,020931 |
| 5-Hydroxy-3-indoleacetic acid (5-HIAA) | 1,1514 | up | 0,021745 |
| CER_Ceramide (d18:2,C23:0) | 0,8822 | down | 0,025435 |
| SM_Sphingomyelin (d18:1,C21:0) | 0,925 | down | 0,026263 |
| Nervonic acid (C24:cis[15]1) | 0,9114 | down | 0,026336 |
| Phenylalanine | 1,0625 | up | 0,0265 |
| Phosphatidylcholine (C16:1,C18:2) | 0,9229 | down | 0,030568 |
| SM_Sphingomyelin (d18:2,C18:1) | 0,9133 | down | 0,0313 |
| CER_Ceramide (d17:1,C16:0) | 0,8986 | down | 0,035021 |
| Cryptoxanthin | 0,8091 | down | 0,036128 |
| Fumarate | 1,0483 | up | 0,036755 |
| Tyrosine | 1,0777 | up | 0,038994 |
| CE_Cholesterylester C20:5 | 0,8236 | down | 0,039914 |
| CE_Cholesterylester C18:4 | 0,7902 | down | 0,043667 |
| Malate | 1,1101 | up | 0,046935 |
| SM_Sphingomyelin (d16:1,C20:0) | 0,9095 | down | 0,053287 |
| CER_Ceramide (d17:1,C22:0) | 0,8882 | down | 0,057993 |
| Glycerol-3-phosphate, polar fraction | 1,1093 | up | 0,061765 |
| Uridine | 0,8946 | down | 0,062565 |
| SM_Sphingomyelin (d17:1,C18:0) | 0,9258 | down | 0,072709 |
| Hippuric acid | 0,7791 | down | 0,081397 |
| CER_Ceramide (d18:1,C23:0) | 0,9177 | down | 0,089112 |
| Phosphate, lipid fraction | 0,9505 | down | 0,097734 |

| | | | |
|---|---|---|---|
| (*1): free and from sphingolipids | | | |

**Table 1c: Metabolites of Table 1a which additionally showed a significant difference (p-value < 0.1) between hypertrophic cardiomyopathy (HCMP) patients and healthy controls**

| Metabolite_Name | ratio of median | regulation | p-value |
|---|---|---|---|
| Maltose | 2,1427 | up | 5,39E-11 |
| Cholesterylester C18:2 | 0,7523 | down | 1,99E-06 |
| Cholesterylester C18:1 | 0,7715 | down | 5,23E-05 |
| Taurine | 1,2525 | up | 9,72E-05 |
| TAG (C16:0,C18:2) | 1,2934 | up | 0,00091 |
| Uric acid | 1,1564 | up | 0,000939 |
| TAG (C18:1 ,C18:2) | 1,3302 | up | 0,00099 |
| Glycerol, lipid fraction | 1,3816 | up | 0,001367 |
| TAG (C16:0,C18:1,C18:2) | 1,3509 | up | 0,002192 |
| CE_Cholesterylester C15:0 | 0,8215 | down | 0,002242 |
| SP_Sphingosine-1-phosphate (d17:1) | 0,8497 | down | 0,002442 |
| SP_Sphinganine (d18:0) | 1,2867 | up | 0,002474 |
| SP_Sphingosine (d18:1) | 1,3486 | up | 0,002704 |
| Sarcosine | 1,0901 | up | 0,003105 |
| beta-Carotene | 0,7568 | down | 0,003481 |
| Cysteine | 1,0924 | up | 0,003905 |
| Tricosanoic acid (C23:0) | 0,8682 | down | 0,004041 |
| TAG (C16:0,C16:1) | 1,3303 | up | 0,004263 |
| Eicosenoic acid (C20:cis[11]1) | 1,2145 | up | 0,005339 |
| Isoleucine | 1,1098 | up | 0,005399 |
| Sphingomyelin (d18:1,C23:0) | 0,926 | down | 0,005483 |
| SM_Sphingomyelin (d18:2,C23:0) | 0,897 | down | 0,006161 |
| Noradrenaline (Norepinephrine) | 1,2232 | up | 0,006926 |
| Lysophosphatidylcholine (C17:0) | 0,8834 | down | 0,008806 |
| Testosterone | 0,8292 | down | 0,009379 |
| TAG (C18:2,C18:2) | 1,2656 | up | 0,009482 |
| Isocitrate | 1,1189 | up | 0,011414 |
| SM_Sphingomyelin (d17:1,C24:0) | 0,885 | down | 0,011423 |
| SM_Sphingomyelin (d17:1,C23:0) | 0,8387 | down | 0,011783 |
| Zeaxanthin | 0,8283 | down | 0,012366 |
| SM_Sphingomyelin (d16:1,C23:0) | 0,869 | down | 0,014315 |
| Cryptoxanthin | 0,7778 | down | 0,018169 |
| Erythrol | 1,121 | up | 0,023299 |
| CER_Ceramide (d17:1,C23:0) | 0,8563 | down | 0,030171 |
| Cholesterylester C16:0 | 0,8446 | down | 0,030834 |
| SM_Sphingomyelin (d17:1,C22:0) | 0,9062 | down | 0,032352 |
| SM_Sphingomyelin (d18:1,C21:0) | 0,9242 | down | 0,032429 |
| SM_Sphingomyelin (d16:1,C21:0) | 0,8795 | down | 0,034803 |
| Glucose | 1,0597 | up | 0,035437 |
| Glutamate | 1,1813 | up | 0,036213 |
| Fumarate | 1,0499 | up | 0,03758 |
| SM_Sphingomyelin (d17:1,C20:0) | 0,9101 | down | 0,039401 |
| CE_Cholesterylester C14:0 | 0,8974 | down | 0,044159 |
| Cystine | 1,1237 | up | 0,044881 |
| 8-Hydroxyeicosatetraenoic acid (C20:trans[5]cis[9,11,14]4) (8-HETE) | 1,1957 | up | 0,047092 |
| 1-Hydroxy-2-amino-(cis,trans)-3,5-octadecadiene (from sphingolipids) | 0,9003 | down | 0,047207 |
| Uridine | 0,8827 | down | 0,047309 |
| Sorbitol | 1,2852 | up | 0,048213 |
| SM_Sphingomyelin (d18:1,C14:0) | 0,9258 | down | 0,049457 |
| Elaidic acid (C18:trans[9]1) | 1,6069 | up | 0,05134 |
| SM_Sphingomyelin (d18:2,C21:0) | 0,9165 | down | 0,052457 |
| Aspartate | 1,0842 | up | 0,056222 |
| Coenzyme Q10 | 1,1425 | up | 0,068217 |
| CER_Ceramide (d18:1,C18:0) | 1,1056 | up | 0,070545 |
| SM_Sphingomyelin (d17:1,C16:0) | 0,9289 | down | 0,07279 |
| SM_Sphingomyelin (d18:2,C23:1) | 0,9224 | down | 0,073683 |
| Lactaldehyde | 1,0822 | up | 0,078804 |
| Pseudouridine | 1,0653 | up | 0,082343 |
| Hippuric acid | 0,7733 | down | 0,083253 |
| SM_Sphingomyelin (d18:1,C23:1) | 0,9341 | down | 0,088944 |
| CER_Ceramide (d17:1,C24:0) | 0,8949 | down | 0,091594 |
| Glucose-1-phosphate | 1,0739 | up | 0,091687 |
| SM_Sphingomyelin (d18:2,C24:0) | 0,933 | down | 0,092261 |

**Table 2: Metabolites with a significant difference (p-value < 0.05) in exercise-induced change between CHF and control**

| Metabolite | ratio of median | regulation | p-value |
|---|---|---|---|
| Glutamate | 0.724 | down | 0.000274 |
| Hypoxanthine | 0.448 | down | 0.000276 |
| Adrenaline (Epinephrine) | 0.439 | down | 0.001258 |
| Lactate | 0.612 | down | 0.005556 |
| Indole-3-lactic acid | 1.198 | up | 0.007027 |
| Threonic acid | 1.160 | up | 0.018026 |
| Cholestenol No 02 | 0.906 | down | 0.022576 |
| alpha-Tocotrienol | 1.206 | up | 0.028952 |
| Coenzyme Q9 | 1.166 | up | 0.029375 |
| Histidine | 1.083 | up | 0.039156 |
| Phosphatidylcholine (C18:0, C20:4) | 1.008 | up | 0.039198 |
| Lysophosphatidylcholine (C18:1) | 1.027 | up | 0.040233 |

**Table 3: Metabolites with a significant difference (p-value < 0.05) between patients with CHF and healthy controls at the peak of exercise (t1) but not at rest (t0)**

| Time point | t0 | t0 | t0 | t1 | t1 | t1 |
|---|---|---|---|---|---|---|
| Metabolite | ratio of median | regulation | p-value | ratio of median | regulation | p-value |
| Lactate | 1.149 | up | 0.161549 | 0.705 | down | 0.015456 |
| Citrate | 1.118 | up | 0.256634 | 1.132 | up | 0.040482 |

**Table 4a: Metabolites with a significant difference (p-value < 0.05) between patients with CHF (dilated cardiomyopathy) with NYHA score 1 and healthy controls**

| Metabolite | ratio of median | regulation | p-value |
|---|---|---|---|
| Mannose | 2.168 | up | 0.000025 |
| Lysophosphatidylcholine (C18:2) | 0.699 | down | 0.000748 |
| Adrenaline (Epinephrine) | 2.411 | up | 0.004448 |
| Hypoxanthine | 1.779 | up | 0.004996 |
| Phosphatidylcholine (C18:0, C18:2) | 1.022 | up | 0.012486 |
| Glucose | 1.271 | up | 0.014916 |
| Phosphate (inorganic and from organic phosphates) | 0.793 | down | 0.015030 |
| Cortisol | 1.340 | up | 0.017261 |
| Phosphatidylcholine (C18:0, C22:6) | 1.239 | up | 0.017614 |
| 2-Hydroxybutyrate | 1.810 | up | 0.019583 |
| Corticosterone | 1.293 | up | 0.019642 |
| Androstenedione | 1.785 | up | 0.035365 |
| Glutamate | 1.333 | up | 0.039299 |
| Pentadecanol | 0.581 | down | 0.044212 |
| Maltose | 1,7858 | up | 8,3846E-06 |
| CE_Cholesterylester C15:0 | 0,7215 | down | 1,073E-05 |
| Cholesterylester C18:2 | 0,7456 | down | 1,7406E-05 |
| SM_Sphingomyelin (d17:1,C24:0) | 0,7957 | down | 2,6209E-05 |
| Noradrenaline (Norepinephrine) | 1,4153 | up | 5,5355E-05 |
| myo-Inositol | 1,1987 | up | 6,44E-05 |
| SM_Sphingomyelin (d17:1,C23:0) | 0,731 | down | 8,1995E-05 |
| SM_Sphingomyelin (d17:1,C22:0) | 0,8196 | down | 0,00013927 |
| Sorbitol | 1,7458 | up | 0,00014037 |
| Normetanephrine | 1,5039 | up | 0,0001699 |
| Isocitrate | 1,2084 | up | 0,00019135 |
| SM_Sphingomyelin (d18:1,C23:0) | 0,8716 | down | 0,00026783 |
| Ornithine | 1,1704 | up | 0,00037428 |
| Erythrol | 1,2249 | up | 0,00040476 |
| Sarcosine | 1,1251 | up | 0,00042563 |
| Cystine | 1,2636 | up | 0,00044298 |
| Testosterone | 0,7586 | down | 0,00086625 |
| CE_Cholesterylester C14:0 | 0,8093 | down | 0,0008742 |
| Uridine | 0,7862 | down | 0,00092815 |
| SM_Sphingomyelin (d18:1,C14:0) | 0,8622 | down | 0,00104019 |
| Lignoceric acid (C24:0) | 0,8223 | down | 0,00134372 |
| Tricosanoic acid (C23:0) | 0,8376 | down | 0,00139431 |
| 1-Hydroxy-2-amino-(cis,trans)-3,5-octadecadiene (from sphingolipids) | 0,8262 | down | 0,00145507 |
| SM_Sphingomyelin (d16:1,C24:0) | 0,7694 | down | 0,00146283 |
| Urea | 1,2149 | up | 0,0015119 |
| beta-Carotene | 0,7083 | down | 0,00164813 |
| Tyrosine | 1,1473 | up | 0,001792 |
| Behenic acid (C22:0) | 0,8547 | down | 0,00192144 |
| alpha-Ketoglutarate | 1,218 | up | 0,00195906 |
| SM_Sphingomyelin (d16:1,C23:0) | 0,8262 | down | 0,00281307 |
| Taurine | 1,2111 | up | 0,00288466 |
| SM_Sphingomyelin (d18:1,C24:0) | 0,8827 | down | 0,0032925 |
| 3-Methoxytyrosine | 1,259 | up | 0,00371589 |
| Lysophosphatidylcholine (C17:0) | 0,8552 | down | 0,00392246 |
| SM_Sphingomyelin (d18:2,C23:0) | 0,8797 | down | 0,00428746 |
| CER_Ceramide (d18:2,C14:0) | 0,8188 | down | 0,00445012 |
| SM_Sphingomyelin (d17:1,C16:0) | 0,8763 | down | 0,00489531 |
| Cholesta-2,4,6-triene | 0,8657 | down | 0,00545382 |
| SM_Sphingomyelin (d18:2,C24:0) | 0,8781 | down | 0,00576839 |
| Phenylalanine | 1,0947 | up | 0,00620035 |
| Cysteine | 1,1 | up | 0,00624402 |
| SM_Sphingomyelin (d16:1,C22:0) | 0,8502 | down | 0,00665211 |
| Uric acid | 1,1441 | up | 0,00696304 |
| CER_Ceramide (d17:1,C24:0) | 0,8237 | down | 0,00931215 |
| Glucose-1-phosphate | 1,1388 | up | 0,00940813 |
| CE_Cholesterylester C22:5 | 0,8609 | down | 0,0095955 |
| CE_Cholesterylester C16:2 | 0,8095 | down | 0,00966362 |
| Dehydroepiandrosterone sulfate | 0,6524 | down | 0,00995067 |
| Glycerol-3-phosphate, polar fraction | 1,1886 | up | 0,00997307 |
| Isoleucine | 1,1158 | up | 0,0102759 |
| SM_Sphingomyelin (d17:1,C20:0) | 0,8765 | down | 0,01056663 |
| CER_Ceramide (d18:1,C14:0) | 0,852 | down | 0,01059946 |
| Cholesterol, total | 0,9069 | down | 0,01060847 |
| SM_Sphingomyelin (d18:1,C22:0) | 0,8438 | down | 0,01179659 |
| Linoleic acid (C18:cis[9,12]2) | 0,8487 | down | 0,01208761 |
| threo-Sphingosine (*1) | 0,8906 | down | 0,01352672 |
| SM_Sphingomyelin (d17:1,C24:1) | 0,9005 | down | 0,01479843 |
| CE_Cholesterylester C16:3 | 0,8114 | down | 0,01621643 |
| CE_Cholesterylester C14:1 | 0,7197 | down | 0,01779781 |
| Cholesterylester C18:1 | 0,837 | down | 0,01841802 |
| scyllo-Inositol | 1,2605 | up | 0,02009089 |
| CE_Cholesterylester C22:6 | 0,8245 | down | 0,02009893 |
| Pseudouridine | 1,0972 | up | 0,02576962 |
| CER_Ceramide (d17:1,C23:0) | 0,8359 | down | 0,02705684 |
| erythro-C16-Sphingosine | 0,8592 | down | 0,02915249 |
| Eicosenoic acid (C20:cis[11]1) | 1,1968 | up | 0,02965701 |
| SP_Sphinganine (d18:0) | 1,2368 | up | 0,03058449 |
| Isopalmitic acid (C16:0) | 0,8326 | down | 0,03139525 |
| Cholesta-2,4-dien | 0,8837 | down | 0,03222468 |
| Lysophosphatidylcholine (C18:0) | 0,8999 | down | 0,03342501 |
| Phosphatidylcholine (C16:1,C18:2) | 0,9093 | down | 0,03389605 |
| Cholesterylester C16:0 | 0,8258 | down | 0,03509819 |
| TAG (C16:0,C18:2) | 1,2113 | up | 0,03532712 |
| SM_Sphingomyelin (d18:2,C22:0) | 0,8964 | down | 0,03540009 |
| CER_Ceramide (d17:1,C16:0) | 0,8814 | down | 0,03839909 |
| Glycerol, lipid fraction | 1,2796 | up | 0,03879761 |
| CE_Cholesterylester C18:3 | 0,8253 | down | 0,04166858 |
| 5-Oxoproline | 1,0601 | up | 0,04385594 |
| CE_Cholesterylester C22:4 | 0,8749 | down | 0,04444786 |
| Serine, lipid fraction | 1,2253 | up | 0,046845 |
| 5-O-Methylsphingosine (*1) | 0,8943 | down | 0,04788647 |
| TAG (C16:0,C18:1,C18:2) | 1,2557 | up | 0,04838256 |
| SP_Sphingosine (d18:1) | 1,2602 | up | 0,04924965 |

| | | | |
|---|---|---|---|
| (*1): free and from sphingolipids | | | |

**Table 4b: Metabolites of Table 4a which additionally showed a significant difference (p-value < 0.1) between ischemic cardiomyopathy (ICMP) patients with NYHA score 1 and healthy controls**

| Metabolite_Name | ratio of median | regulation | p-value |
|---|---|---|---|
| Cholesterylester C18:2 | 0,6118 | down | 1,7191E-12 |
| SM_Sphingomyelin (d18:1,C14:0) | 0,7778 | down | 3,7018E-08 |
| Sorbitol | 2,0982 | up | 4,3743E-07 |
| SM_Sphingomyelin (d18:2,C23:0) | 0,8125 | down | 4,0589E-06 |
| SM_Sphingomyelin (d17:1,C23:0) | 0,7067 | down | 1,1938E-05 |
| CE_Cholesterylester C15:0 | 0,7269 | down | 1,472E-05 |
| SM_Sphingomyelin (d18:1,C23:0) | 0,8512 | down | 1,8295E-05 |
| TAG (C16:0,C18:2) | 1,453 | up | 1,8737E-05 |
| Cholesterylester C18:1 | 0,7343 | down | 1,939E-05 |
| Tricosanoic acid (C23:0) | 0,7919 | down | 2,5541E-05 |
| 1-Hydroxy-2-amino-(cis,trans)-3,5-octadecadiene (from sphingolipids) | 0,7813 | down | 3,8025E-05 |
| Cholesterol, total | 0,8603 | down | 3,8932E-05 |
| TAG (C16:0,C18:1,C18:2) | 1,572 | up | 4,2286E-05 |
| SM_Sphingomyelin (d17:1,C16:0) | 0,8268 | down | 5,0421E-05 |
| CE_Cholesterylester C14:0 | 0,785 | down | 6,3189E-05 |
| beta-Carotene | 0,6577 | down | 0,00012609 |
| threo-Sphingosine (*1) | 0,8433 | down | 0,00014084 |
| Cholesta-2,4-dien | 0,8112 | down | 0,00014837 |
| Lysophosphatidylcholine (C17:0) | 0,8168 | down | 0,00018589 |
| Glucose | 1,1224 | up | 0,00021581 |
| Glutamate | 1,3974 | up | 0,00024219 |
| SM_Sphingomyelin (d17:1,C24:0) | 0,8216 | down | 0,00026196 |
| Lignoceric acid (C24:0) | 0,8114 | down | 0,00031083 |
| SM_Sphingomyelin (d16:1,C23:0) | 0,7977 | down | 0,00038589 |
| Phosphatidylcholine (C18:0,C18:2) | 1,0177 | up | 0,00040589 |
| SM_Sphingomyelin (d18:2,C24:0) | 0,8492 | down | 0,00049962 |
| 5-O-Methylsphingosine (*1) | 0,8249 | down | 0,0006501 |
| SM_Sphingomyelin (d17:1,C22:0) | 0,8428 | down | 0,00094804 |
| Cystine | 1,2438 | up | 0,00096287 |
| Taurine | 1,2299 | up | 0,00120903 |
| Glucose-1-phosphate | 1,1646 | up | 0,00135235 |
| SM_Sphingomyelin (d17:1,C24:1) | 0,8718 | down | 0,00137508 |
| Glycerol, lipid fraction | 1,4351 | up | 0,00147588 |
| Behenic acid (C22:0) | 0,8594 | down | 0,00159109 |
| SM_Sphingomyelin (d16:1,C24:0) | 0,7739 | down | 0,00173184 |
| Isocitrate | 1,1685 | up | 0,00194376 |
| Cysteine | 1,1133 | up | 0,0019666 |
| 3-Methoxytyrosine | 1,2542 | up | 0,00284987 |
| CER_Ceramide (d18:1,C14:0) | 0,8291 | down | 0,00290481 |
| erythro-C16-Sphingosine | 0,8147 | down | 0,00311066 |
| Linoleic acid (C18:cis[9,12]2) | 0,8385 | down | 0,00451392 |
| Maltose | 1,4331 | up | 0,00496723 |
| Adrenaline (Epinephrine) | 1,5012 | up | 0,00542671 |
| SM_Sphingomyelin (d18:2,C22:0) | 0,8703 | down | 0,00727388 |
| Lysophosphatidylcholine (C18:2) | 0,8695 | down | 0,00744797 |
| Normetanephrine | 1,3345 | up | 0,00759363 |
| SM_Sphingomyelin (d18:1,C24:0) | 0,8937 | down | 0,0076034 |
| Cholesterylester C16:0 | 0,7884 | down | 0,00805685 |
| Eicosenoic acid (C20:cis[11]1) | 1,2302 | up | 0,00826261 |
| Cholesta-2,4,6-triene | 0,8784 | down | 0,00837711 |
| CE_Cholesterylester C22:5 | 0,8605 | down | 0,00891577 |
| Dehydroepiandrosterone sulfate | 0,6661 | down | 0,00966052 |
| Pseudouridine | 1,1119 | up | 0,01037548 |
| CE_Cholesterylester C22:4 | 0,8457 | down | 0,01129319 |
| CE_Cholesterylester C14:1 | 0,7165 | down | 0,01133041 |
| Lysophosphatidylcholine (C18:0) | 0,8831 | down | 0,01177707 |
| Uric acid | 1,1327 | up | 0,01187686 |
| SM_Sphingomyelin (d18:1,C22:0) | 0,8458 | down | 0,01247557 |
| Testosterone | 0,8204 | down | 0,01585512 |
| CER_Ceramide (d17:1,C23:0) | 0,8278 | down | 0,02004195 |
| SM_Sphingomyelin (d16:1,C22:0) | 0,875 | down | 0,0242648 |
| Noradrenaline (Norepinephrine) | 1,2117 | up | 0,02471946 |
| CE_Cholesterylester C16:2 | 0,8476 | down | 0,03239574 |
| 5-Oxoproline | 1,0599 | up | 0,03394216 |
| alpha-Ketoglutarate | 1,1326 | up | 0,03826297 |
| CER_Ceramide (d17:1,C24:0) | 0,8583 | down | 0,04035007 |
| Isopalmitic acid (C16:0) | 0,8489 | down | 0,04227044 |
| CE_Cholesterylester C18:3 | 0,8356 | down | 0,04430951 |
| CE_Cholesterylester C22:6 | 0,8484 | down | 0,04599329 |
| SM_Sphingomyelin (d17:1,C20:0) | 0,9092 | down | 0,06237979 |
| Isoleucine | 1,0817 | up | 0,06356105 |
| Tyrosine | 1,083 | up | 0,06681343 |
| Ornithine | 1,0775 | up | 0,07275574 |
| Phosphatidylcholine (C16:1,C18:2) | 0,9234 | down | 0,0730987 |
| Mannose | 1,1099 | up | 0,07913279 |
| myo-Inositol | 1,08 | up | 0,08438868 |

| | | | |
|---|---|---|---|
| (*1): free and from sphingolipids | | | |

**Table 4c: Metabolites of Table 4a which additionally showed a significant difference (p-value < 0.1) between HCMP patients with NYHA 1 scores and healthy controls**

| Metabolite_Name | ratio of median | regulation | p-value |
|---|---|---|---|
| Maltose | 2,3774 | up | 9,1877E-11 |
| Cholesterylester C18:2 | 0,7422 | down | 1,5121E-05 |
| Taurine | 1,3057 | up | 4,2799E-05 |
| Cholesterylester C18:1 | 0,7566 | down | 0,00017957 |
| Isoleucine | 1,1583 | up | 0,00067494 |
| TAG (C16:0,C18:2) | 1,3413 | up | 0,00106071 |
| Sarcosine | 1,1148 | up | 0,00123586 |
| SP_Sphinganine (d18:0) | 1,3661 | up | 0,00126025 |
| SP_Sphingosine (d18:1) | 1,4493 | up | 0,00135359 |
| TAG (C16:0,C18:1,C18:2) | 1,4301 | up | 0,00163138 |
| CE_Cholesterylester C15:0 | 0,814 | down | 0,00544571 |
| SM_Sphingomyelin (d18:1,C23:0) | 0,9016 | down | 0,00613976 |
| Tricosanoic acid (C23:0) | 0,8591 | down | 0,00645307 |
| SM_Sphingomyelin (d18:2,C23:0) | 0,8856 | down | 0,00715908 |
| Glycerol, lipid fraction | 1,3643 | up | 0,00800466 |
| Eicosenoic acid (C20:cis[11]1) | 1,2284 | up | 0,01113831 |
| SM_Sphingomyelin (d17:1,C23:0) | 0,8234 | down | 0,01457624 |
| Uric acid | 1,1278 | up | 0,01663987 |
| beta-Carotene | 0,7703 | down | 0,01772396 |
| Serine, lipid fraction | 1,2593 | up | 0,02396587 |
| Testosterone | 0,8387 | down | 0,03444244 |
| CE_Cholesterylester C22:5 | 0,8857 | down | 0,03705511 |
| Noradrenaline (Norepinephrine) | 1,1939 | up | 0,03929456 |
| CE_Cholesterylester C22:4 | 0,8728 | down | 0,04240014 |
| 1-Hydroxy-2-amino-(cis,trans)-3,5-octadecadiene (from sphingolipids) | 0,8851 | down | 0,04256896 |
| Uridine | 0,8639 | down | 0,04452619 |
| Glutamate | 1,199 | up | 0,04801547 |
| Lysophosphatidylcholine (C17:0) | 0,8984 | down | 0,04926739 |
| SM_Sphingomyelin (d16:1,C23:0) | 0,8842 | down | 0,05494844 |
| Cholesterylester C16:0 | 0,8449 | down | 0,06302395 |
| SM_Sphingomyelin (d18:1,C14:0) | 0,9196 | down | 0,06434119 |
| SM_Sphingomyelin (d17:1,C22:0) | 0,9084 | down | 0,0655624 |
| SM_Sphingomyelin (d18:2,C24:0) | 0,9168 | down | 0,06627783 |
| Erythrol | 1,112 | up | 0,06717477 |
| Isocitrate | 1,097 | up | 0,06783798 |
| SM_Sphingomyelin (d17:1,C20:0) | 0,9104 | down | 0,06992485 |
| SM_Sphingomyelin (d17:1,C24:0) | 0,9103 | down | 0,08265925 |
| CER_Ceramide (d18:2,C14:0) | 0,8865 | down | 0,08795584 |

**Table 5: Metabolites with a significant difference (p-value < 0.05) in exercise-induced change between CHF with NYHA score 1 and control**

| Metabolite | ratio of median | regulation | p-value |
|---|---|---|---|
| Glutamate | 0.720 | down | 0.025093 |
| Hypoxanthine | 0.407 | down | 0.034843 |
| Phosphatidylcholine (C18:0, C20:4) | 1.011 | up | 0.048864 |

**Table 6: Metabolites with a significant difference (p-value < 0.05) between patients with CHF with NYHA score I at the peak of exercise (t1) but not at rest (t0)**

| Time point | t0 | t0 | t0 | t1 | t1 | t1 |
|---|---|---|---|---|---|---|
| Parameter | ratio of median | regulation | p-value | ratio of median | regulation | p-value |
| Phosphatidylcholine (C18:0, C20:4) | 1.0359 00639 | up | 0.33999 4 | 1.0542745 85 | up | 0.04949 2 |

**Table 7: Chemical/physical properties of selected analytes. These biomarkers are characterized herein by chemical and physical properties.**

| Metabolite | Fragmentation pattern (GC-MS) and description |
|---|---|
| Glycerol phosphate, lipid fraction | Glycerol phosphate, lipid fraction represents the sum parameter of metabolites containing a glycerol-2-phosphate or a glycerol-3-phosphate moiety and being present in the lipid fraction after extraction and separation of the extract into a polar and a lipid fraction. |
| 3-O-Methylsphingosine | 3-O-Methylsphingosine exhibits the following characteristic ionic fragments if detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 204 (100), 73 (18), 205 (16), 206 (7), 354 (4), 442 (1). |
| 5-O-Methylsphingosine | 5-O-Methylsphingosine exhibits the following characteristic ionic fragments if detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 250 (100), 73 (34), 251 (19), 354 (14), 355 (4), 442 (1). |
| Phosphatidylcholine No 02 | Phosphatidylcholine No 02 represents the sum parameter of phosphatidylcholines. It exhibits the following characteristic ionic species when detected with LC/MS, applying electro-spray ionization (ESI) mass spectrometry: mass-to-charge ratio (m/z) of the positively charged ionic species is 808.4 (+/- 0.5). |
| TAG (C16:0,C16:1) | TAG (C16:0,C16:1) represents the sum parameter of triacylglycerides containing the combination of a C16:0 fatty acid unit and a C16:1 fatty acid unit. It exhibits the following characteristic ionic species when detected with LC/MS, applying electro-spray ionization (ESI) mass spectrometry: mass-to-charge ratio (m/z) of the positively charged ionic species is 549.6 (+/- 0.5). |
| TAG (C16:0,C18:2) | TAG (C16:0,C18:2) represents the sum parameter of triacylglycerides containing the combination of a C16:0 fatty acid unit and a C18:2 fatty acid unit. It exhibits the following characteristic ionic species when detected with LC/MS, applying electro-spray ionization (ESI) mass spectrometry: mass-to-charge ratio (m/z) of the positively charged ionic species is 575.6 (+/- 0.5). |
| TAG (C18:1,C18:2) | TAG (C18:1,C18:2) represents the sum parameter of triacylglycerides containing the combination of a C18:1 fatty acid unit and a C18:2 fatty acid unit. It exhibits the following characteristic ionic species when detected with LC/MS, applying electro-spray ionization (ESI) mass spectrometry: mass-to-charge ratio (m/z) of the positively charged ionic species is 601.6 (+/- 0.5). |
| TAG (C18:2,C18:2) | TAG (C18:2,C18:2) represents the sum parameter of triacylglycerides containing the combination of two C18:2 fatty acid units. It exhibits the following characteristic ionic species when detected with LC/MS, applying electro-spray ionization (ESI) mass spectrometry: mass-to-charge ratio (m/z) of the positively charged ionic species is 599.6 (+/- 0.5). |
| Cholestenol No 02 | Cholestenol No 02 represents a Cholestenol isomer. It exhibits the following characteristic ionic fragments if detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 143 (100), 458 (91), 73 (68), 81 (62), 95 (36), 185 (23), 327 (23), 368 (20), 255 (15), 429 (15). |

Diagnostic and risk marker for diabetes according to WO2012/085890:

**Table 8: Diabetes biomarker glyoxylate**

| | | | | |
|---|---|---|---|---|
| glyoxylate | Diagnostic question | Direction | p-value | Ratio |
| | Diabetes vs. healthy | Up | 0.011 | 1.23 |
| | Non-healthy (risk & diabetes) vs. healthy subjects | Up | 0.017 | 1.13 |
| | Risk by OGTT vs. healthy | Up | 0.024 | 1.16 |
| | All risk subjects vs. healthy | Up | 0.052 | 1.11 |
| | Glucose-based comparison positive (diabetes and risk subjects) vs. neg negative (healthy controls, (quantile thresholds with gap in glucose concentration) | Up | 0.0026 | 1.19 |
| | Correlation with numeric HbA1c | Up | 0.056 | 1.051 |
| | HbA1c-based comparison pos vs. neg (standard thresholds with gap) | Up | 0.036 | 1.15 |
| | HbA1c-based comparison pos vs. neg (quantile thresholds with gap) | Up | 0.039 | 1.13 |
| | Diabetes vs. IFG | Up | 0.095 | 1.15 |
| | IFG+IGT vs. healthy | Up | 0.0043 | 1.23 |
| | Diabetes vs. IGT | Up | 0.095 | 1.20 |
| | Diabetes detectable by fasting glucose vs. healthy | Up | 0.0082 | 1.32 |

Diagnostic markers for diabetes according to WO2007/110357:

**Table 9: New Diabetes-specific metabolites determined on entire dataset. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided.**

| Chemical name | regulation | fold change | p.t |
|---|---|---|---|
| 1,5-Anhydrosorbitol | down | 0,83 | 1,68E-10 |
| Eicosenoic acid (C20:1) | up | 1,23 | 3,68E-09 |
| Erythrol | up | 1,17 | 1,87E-08 |
| Ribonic acid | up | 1,12 | 0,000207352 |
| Tricosanoic acid (C23:0) | down | 0,91 | 0,000690021 |
| Pentadecanol | up | 1,14 | 0,002821548 |
| Campesterol | down | 0,92 | 0,008032527 |
| Maleic Acid | down | 0,93 | 0,012630545 |
| Melissic Acid (C30:0) | down | 0,97 | 0,032299205 |

**Table 10: New Diabetes-specific metabolites determined on age-matched males. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided.**

| Chemical name | regulation | fold change | p.t |
|---|---|---|---|
| 1,5-Anhydrosorbitol | down | 0,715966162 | 5,46E-07 |
| Eicosenoic acid (C20:1) | up | 1,289836715 | 0,00169478 |
| Pentadecanol | up | 1,215689075 | 0,029197314 |

**Table 11: New Diabetes-specific metabolites determined on age-matched females. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided.**

| Chemical name | regulation | fold change | p.t |
|---|---|---|---|
| Eicosenoic acid (C20:1) | up | 1,179797938 | 0,001492544 |
| Campesterol | down | 0,808075198 | 0,003629138 |
| Tricosanoic acid (C23:0) | down | 0,894095758 | 0,013812625 |
| Ribonic acid | up | 1,138360459 | 0,01522522 |
| Erythrol | up | 1,129463926 | 0,033964934 |

**Table 12: New Diabetes-specific metabolites combined from Table 1-3. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided.**

| Chemical name | regulation | fold change | p.t |
|---|---|---|---|
| 1,5-Anhydrosorbitol | down | 0,829793095 | 1,68E-10 |
| Eicosenoic acid (C20:1) | up | 1,232521755 | 3,68E-09 |
| Erythrol | up | 1,165086499 | 1,87E-08 |
| Ribonic acid | up | 1,123283244 | 0,000207352 |
| Tricosanoic acid (C23:0) | down | 0,914819475 | 0,000690021 |
| Pentadecanol | up | 1,137229303 | 0,002821548 |
| Campesterol | down | 0,808075198 | 0,003629138 |
| Maleic Acid | down | 0,925831953 | 0,012630545 |
| Melissic Acid (C30:0) | down | 0,967955786 | 0,032299205 |

**Table 13: Diabetes-specific metabolites determined on entire dataset. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided. The trivial finding of significantly altered Glucose levels of diabetes patients relative to control subjects was excluded from the table.**

| Chemical name | regulation | fold change | p.t |
|---|---|---|---|
| Ascorbic acid | up | 1,46 | 3,36E-57 |
| Mannose | up | 1,49 | 1,73E-42 |
| Valine | up | 1,20 | 5,67E-21 |
| Isoleucine | up | 1,23 | 4,91E-20 |
| Leucine | up | 1,19 | 7,13E-18 |
| Uric acid | up | 1,22 | 3,51E-17 |
| Cysteine | up | 1,27 | 6,53E-15 |
| putative DAG (C18:1,C18:2 or C18:0,C18:3) | up | 1,35 | 1,65E-14 |
| Pyruvate | up | 1,43 | 1,08E-13 |
| Glycerol, lipid fraction | up | 1,36 | 2,60E-13 |
| Alanine | up | 1,16 | 9,73E-13 |
| Docosahexaenoic acid (C22:cis[4,7,10,13,16,19]6) | up | 1,35 | 2,92E-12 |
| a-Ketoisocaproic acid | up | 1,36 | 3,71E-12 |
| Tyrosine | up | 1,15 | 3,94E-12 |
| Coenzyme Q10 | up | 1,44 | 4,82E-12 |
| Phenylalanine | up | 1,12 | 4,79E-10 |
| Arachidonic acid (C20:cis-[5,8,11,14]4) | up | 1,18 | 1,03E-09 |
| Palmitic acid (C16:0) | up | 1,16 | 2,25E-09 |
| Glycine | down | 0,88 | 3,11E-07 |
| Methionine | up | 1,12 | 3,97E-07 |
| Eicosapentaenoic acid (C20:cis[5,8,11,14,17]5) | up | 1,40 | 6,24E-07 |
| Proline | up | 1,13 | 8,62E-07 |
| Pantothenic acid | up | 1,15 | 8,71E-07 |
| Stearic acid (C18:0) | up | 1,12 | 1,88E-06 |
| Citrate | up | 1,10 | 2,00E-06 |
| Heptadecanoic acid (C17:0) | up | 1,13 | 3,08E-06 |
| trans-9-Hexadecenoic acid (C16:trans[9]1) | up | 1,23 | 1,01E-05 |
| Urea | up | 1,15 | 1,39E-05 |
| Myristic acid (C14:0) | up | 1,24 | 2,07E-05 |
| trans-4-Hydroxyprolin | up | 1,17 | 3,23E-05 |
| 3-Hydroxybutyric acid | up | 1,29 | 5,88E-05 |
| Malate | up | 1,09 | 7,55E-05 |
| Lignoceric acid (C24:0) | down | 0,92 | 0,000180162 |
| myo-Inositol | up | 1,10 | 0,00026466 |
| Phosphate (inorganic and from organic phosphates) | up | 1,06 | 0,000360853 |
| Glycerol, polar fraction | up | 1,12 | 0,000497516 |
| Lysine | up | 1,09 | 0,001206357 |
| Creatinine | up | 1,12 | 0,004335171 |
| Threonic acid | down | 0,90 | 0,00480835 |
| Succinate | down | 0,93 | 0,005840745 |
| Glyceric acid | down | 0,90 | 0,006088538 |
| Linolenic acid (C18:cis[9,12,15]3) | up | 1,10 | 0,006887601 |
| Lactate | up | 1,10 | 0,007055085 |
| Glycerol-3-Phosphate, polar fraction | up | 1,08 | 0,010395131 |
| Threonine | down | 0,95 | 0,011333993 |
| Phosphate, lipid (Phospholipids) | down | 0,96 | 0,011654865 |
| alpha-Tocopherol | up | 1,15 | 0,01644293 |
| myo-Inositol-2-monophosphate, lipid fraction (myo-Inositolphospholipids) | up | 1,10 | 0,023497772 |
| Linoleic acid (C18:cis[9,12]2) | up | 1,05 | 0,029803521 |
| Cholesterol | down | 0,95 | 0,040018899 |
| Tryptophane | up | 1,04 | 0,044645682 |
| Glutamine | up | 1,08 | 0,048316597 |

**Table 14: Diabetes-specific metabolites determined on age-mached males. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided. The trivial finding of significantly altered Glucose levels of diabetes patients relative to control subjects was excluded from the table.**

| Chemical name | regulation | fold change | p.t |
|---|---|---|---|
| Ascorbic acid | up | 1,484165764 | 4,48E-16 |
| Mannose | up | 1,441573139 | 1,02E-10 |
| Triacylglycerides (containing C16:1, C18:1 or C16:0) | up | 1,241759768 | 5,15E-06 |
| Glycerol, lipid fraction | up | 1,450283984 | 0,000120249 |
| Valine | up | 1,1519912 | 0,000250545 |
| Glycine | down | 0,893625097 | 0,000402058 |
| Uric acid | up | 1,154617325 | 0,000417209 |
| Alanine | up | 1,135942086 | 0,000824962 |
| Isoleucine | up | 1,14342636 | 0,000977933 |
| Leucine | up | 1,122545097 | 0,001040907 |
| a-Ketoisocaproic acid | up | 1,237299055 | 0,001333169 |
| Cysteine | up | 1,185825621 | 0,002788438 |
| trans-9-Hexadecenoic acid (C16:trans[9]1) | up | 1,335554411 | 0,003179817 |
| Palmitic acid (C16:0) | up | 1,154644873 | 0,00355258 |
| Phosphate (inorganic and from organic phosphates) | up | 1,085474184 | 0,003897319 |
| Tyrosine | up | 1,101189829 | 0,006262303 |
| Pantothenic acid | up | 1,150110477 | 0,008641156 |
| Myristic acid (C14:0) | up | 1,347548843 | 0,00904407 |
| Coenzyme Q10 | up | 1,358078148 | 0,010579477 |
| Pyruvate | up | 1,219379362 | 0,01116163 |
| Stearic acid (C18:0) | up | 1,135222404 | 0,01651251 |
| Heptadecanoic acid (C17:0) | up | 1,135873084 | 0,016656669 |
| Arachidonic acid (C20:cis-[5,8,11,14]4) | up | 1,113293751 | 0,017485633 |
| Citrate | up | 1,085160753 | 0,017527845 |
| Threonic acid | down | 0,841572782 | 0,02001934 |
| Threonine | down | 0,92665537 | 0,029210563 |
| Proline | up | 1,103973996 | 0,034468001 |
| Phenylalanine | up | 1,088412821 | 0,035540147 |
| Glycerol, polar fraction | up | 1,146918974 | 0,038229859 |
| Ornithine | down | 0,920136988 | 0,042452599 |
| Malate | up | 1,104999923 | 0,04703203 |

**Table 15: Diabetes-specific metabolites determined on age-mached females. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided. The trivial finding of significantly altered Glucose levels of diabetes patients relative to control subjects was excluded from the table.**

| Chemical name | regulation | fold change | p.t |
|---|---|---|---|
| Ascorbic acid | up | 1,380715922 | 2,95E-15 |
| Mannose | up | 1,462099754 | 2,85E-14 |
| Isoleucine | up | 1,249533174 | 3,91E-10 |
| Valine | up | 1,216130562 | 9,47E-10 |
| Leucine | up | 1,209312876 | 4,11E-09 |
| Uric acid | up | 1,212338486 | 3,68E-07 |
| putative DAG (C18:1,C18:2 or C18:0,C18:3) | up | 1,334873111 | 1,96E-06 |
| Pyruvate | up | 1,422173491 | 2,55E-06 |
| Glycerol, lipid fraction | up | 1,293094601 | 3,32E-06 |
| Cysteine | up | 1,218774727 | 2,91E-05 |
| Alanine | up | 1,151999587 | 3,40E-05 |
| Arachidonic acid (C20:cis-[5,8,11,14]4) | up | 1,184397856 | 4,34E-05 |
| a-Ketoisocaproic acid | up | 1,331702228 | 6,58E-05 |
| Tyrosine | up | 1,140901171 | 7,41E-05 |
| Phenylalanine | up | 1,117874407 | 0,000102016 |
| Palmitic acid (C16:0) | up | 1,151136844 | 0,000163626 |
| Docosahexaenoic acid (C22:cis[4,7,10,13,16,19]6) | up | 1,26073495 | 0,000260771 |
| Glycine | down | 0,865358103 | 0,000565068 |
| Stearic acid (C18:0) | up | 1,111573897 | 0,00072957 |
| Coenzyme Q10 | up | 1,266195595 | 0,000749378 |
| Methionine | up | 1,105511152 | 0,002156394 |
| Proline | up | 1,12556561 | 0,002831665 |
| Citrulline | down | 0,913837925 | 0,004639509 |
| Eicosapentaenoic acid | up | 1,365025845 | 0,005431358 |
| (C20:cis[5,8,11,14,17]5) | | | |
| Phosphate (inorganic and from organic phosphates) | up | 1,081308636 | 0,006424403 |
| Tryptophane | up | 1,072449337 | 0,011971631 |
| 3-Hydroxybutyric acid | up | 1,173601577 | 0,012617371 |
| Heptadecanoic acid (C17:0) | up | 1,101194333 | 0,014202784 |
| trans-9-Hexadecenoic acid (C16:trans[9]1) | up | 1,171432156 | 0,014395605 |
| Lignoceric acid (C24:0) | down | 0,904681793 | 0,014423836 |
| Malate | up | 1,094591121 | 0,019963926 |
| Myristic acid (C14:0) | up | 1,161581037 | 0,022090354 |
| Glycerol, polar fraction | up | 1,112976588 | 0,039329749 |
| trans-4-Hydroxyprolin | up | 1,155965403 | 0,048937139 |

**Table 16: Diabetes-specific metabolites combined from Table 1-3. Metabolites ("CHEMICAL NAME") are sorted according to t-Test p-value ("p.t") starting with most significant findings. Also, fold change values ("Fold-change": mean signal ratios of diabetes patients divided by mean signal ratio of control subjects) and regulation type in diabetes patients ("Kind of regulation": distinguishing whether fold change is above 1 ("up") or below 1 ("down")) are provided. The trivial finding of significantly altered Glucose levels of diabetes patients relative to control subjects was excluded from the table.**

| Chemical name | regulation | fold change | p.t |
|---|---|---|---|
| Ascorbic acid | up | 1,460897562 | 3,36E-57 |
| Mannose | up | 1,49099366 | 1,73E-42 |
| Valine | up | 1,201219187 | 5,67E-21 |
| Isoleucine | up | 1,226340595 | 4,91E-20 |
| Leucine | up | 1,189558225 | 7,13E-18 |
| Uric acid | up | 1,221580228 | 3,51E-17 |
| Cysteine | up | 1,272344952 | 6,53E-15 |
| putative DAG (C18:1,C18:2 or C18:0,C18:3) | up | 1,354261116 | 1,65E-14 |
| Pyruvate | up | 1,428873302 | 1,08E-13 |
| Glycerol, lipid fraction | up | 1,356574719 | 2,60E-13 |
| Alanine | up | 1,1628012 | 9,73E-13 |
| Docosahexaenoic acid (C22:cis[4,7,10,13,16,19]6) | up | 1,351684129 | 2,92E-12 |
| a-Ketoisocaproic acid | up | 1,355419473 | 3,71E-12 |
| Tyrosine | up | 1,147988422 | 3,94E-12 |
| Coenzyme Q10 | up | 1,437313752 | 4,82E-12 |
| Phenylalanine | up | 1,121836648 | 4,79E-10 |
| Arachidonic acid (C20:cis-[5,8,11,14]4) | up | 1,177263087 | 1,03E-09 |
| Palmitic acid (C16:0) | up | 1,157367192 | 2,25E-09 |
| Glycine | down | 0,883191047 | 3,11E-07 |
| Methionine | up | 1,122195372 | 3,97E-07 |
| Eicosapentaenoic acid (C20:cis[5,8,11,14,17]5) | up | 1,403223234 | 6,24E-07 |
| Proline | up | 1,13167844 | 8,62E-07 |
| Pantothenic acid | up | 1,154905329 | 8,71E-07 |
| Stearic acid (C18:0) | up | 1,11726154 | 1,88E-06 |
| Citrate | up | 1,098766652 | 2,00E-06 |
| Heptadecanoic acid (C17:0) | up | 1,13334341 | 3,08E-06 |
| trans-9-Hexadecenoic acid (C16:trans[9]1) | up | 1,231675019 | 1,01E-05 |
| Urea | up | 1,14574428 | 1,39E-05 |
| Myristic acid (C14:0) | up | 1,243213274 | 2,07E-05 |
| trans-4-Hydroxyprolin | up | 1,170068568 | 3,23E-05 |
| 3-Hydroxybutyric acid | up | 1,289932939 | 5,88E-05 |
| Malate | up | 1,094925736 | 7,55E-05 |
| Lignoceric acid (C24:0) | down | 0,917996389 | 0,000180162 |
| myo-Inositol | up | 1,101603199 | 0,00026466 |
| Phosphate (inorganic and from organic phosphates) | up | 1,063347665 | 0,000360853 |
| Glycerol, polar fraction | up | 1,124778954 | 0,000497516 |
| Lysine | up | 1,090319289 | 0,001206357 |
| Creatinine | up | 1,121185726 | 0,004335171 |
| Citrulline | down | 0,913837925 | 0,004639509 |
| Threonic acid | down | 0,899837419 | 0,00480835 |
| Succinate | down | 0,92986853 | 0,005840745 |
| Glyceric acid | down | 0,903105894 | 0,006088538 |
| Linolenic acid (C18:cis[9,12,15]3) | up | 1,095025387 | 0,006887601 |
| Lactate | up | 1,104215189 | 0,007055085 |
| Glycerol-3-Phosphate, polar fraction | up | 1,084629455 | 0,010395131 |
| Threonine | down | 0,95499908 | 0,011333993 |
| Phosphate, lipid (Phospholipids) | down | 0,958528553 | 0,011654865 |
| Tryptophane | up | 1,072449337 | 0,011971631 |
| alpha-Tocopherol | up | 1,14791735 | 0,01644293 |
| myo-Inositol-2-monophosphate, lipid fraction (myo-Inositolphospholipids) | up | 1,097917328 | 0,023497772 |
| Linoleic acid (C18:cis[9,12]2) | up | 1,048610793 | 0,029803521 |
| Cholesterol | down | 0,946204153 | 0,040018899 |
| Ornithine | down | 0,920136988 | 0,042452599 |
| Glutamine | up | 1,075976861 | 0,048316597 |

Diabetes risk markers according to WO2007/110358:

**Table 17: Overall results. Metabolites differing significantly (p < 0,05) between risk groups for Diabetes mellitus type 2 (IFG, IGT and IFG&IGT) and controls (significant main effect "risk", i.e. same regulation type ("up", "down") in males and females.). Metabolites sorted by p-value. [IFG = Impaired Fasting Glucose; IGT = Impaired Glucose Tolerance; IFG&IGT: patients having both IFG and IGT]**

| metabolite | regulation | risk_group |
|---|---|---|
| cryptoxanthin | down | IGT |
| 2-hydroxy-palmitic acid | up | IFG |
| triacylglyceride (C16:0,C18:1,C18:2) | | IGT |
| gondoic acid | up | IGT |
| tricosanoic acid | down | IFG&IGT |
| 5-Oxoproline | up | IFG |

**Table 18: Metabolites differing specifically between male controls and male patients from risk groups for Diabetes. Metabolites differing significantly (p < 0,05) with regard to interaction risk-gender, i.e. differently regulated in males and females with regard to risk for Diabetes mellitus type 2 (IFG, IGT and IFG&IGT) and controls. Metabolites sorted by p-value. [IFG = Impaired Fasting Glucose; IGT = Impaired Glucose Tolerance; IFG&IGT: patients having both IFG and IGT]**

| metabolite | reg_male | risk_group |
|---|---|---|
| diacylglyceride (C18:1,C18:2) | down | IGT |
| triacylglyceride (C16:0,C18:2,C18:2) | down | IGT |
| triacylglyceride (C16:0,C18:1 ,C18:2) | down | IFG |

**Table 19: Overall results. Metabolites differing significantly (p < 0,05) between risk groups for Diabetes mellitus type 2 (IFG, IGT and IFG&IGT) and controls (significant main effect "risk", i.e. same regulation type ("up", "down") in males and females.). Metabolites sorted by p-value. [IFG = Impaired Fasting Glucose; IGT = Impaired Glucose Tolerance; IFG&IGT: patients having both IFG and IGT]**

| metabolite | regulation | risk_group |
|---|---|---|
| lactate | up | IFG |
| alpha-ketoisocaproic acid | down | IGT |
| glucose | up | IFG&IGT |
| methionine | down | IGT |
| mannose | up | IFG&IGT |
| 3-hydroxybutyric acid | up | IGT |
| leucine | up | IGT |
| uric acid | up | IFG |
| threonic acid | up | IFG |
| beta-carotene | down | IFG&IGT |
| ascorbic acid | up | IFG&IGT |
| glycine | down | IGT |
| triacylglycerides | down | IFG |
| lactate | up | IGT |
| phospholipids | up | IGT |
| creatinine | down | IGT |
| glutamate | up | IFG |
| alpha-ketoisocaproic acid | down | IFG&IGT |
| triacylglycerides | up | IGT |
| valine | up | IGT |
| malate | up | IFG |
| alpha-ketoisocaproic acid | down | IFG |
| isoleucine | up | IGT |
| succinate | up | IFG |
| glucose-1-phosphate | up | IFG&IGT |
| valine | up | IFG&IGT |
| eicosapentaenoic acid (C20:cis[5,8,11,14,17]5) | down | IFG&IGT |
| phospholipids | up | IFG |
| uric acid | up | IFG&IGT |
| citrate | up | IGT |
| aspargine | down | IFG&IGT |
| methionine | down | IFG |
| glutamine | down | IGT |
| palmitic acid | up | IGT |
| tryptophane | down | IFG&IGT |
| alanine | up | IGT |
| glutamate | up | IGT |
| citrulline | down | IGT |
| cholestenol | down | IFG&IGT |
| threonine | down | IGT |
| ornithine | up | IFG |
| arginine | down | IGT |
| mannose | up | IFG |
| 3-hydroxybutyric acid | up | IFG&IGT |
| glutamine | down | IFG |
| pregnenolone sulfate | up | IFG&IGT |
| glyceric acid | up | IGT |
| folate | up | IFG |
| malate | up | IGT |
| beta-carotene | down | IFG |
| leucine | up | IFG |
| glutamine | down | IFG&IGT |
| alpha-tocopherol | up | IFG&IGT |
| myo-inositol | up | IFG |
| stearic acid | up | IGT |
| glycerol-3-phosphate | up | IFG |
| beta-carotene | down | IGT |

**Table 20: Metabolites differing specifically between male controls and male patients from risk groups for Diabetes. Metabolites differing significantly (p < 0,05) with regard to interaction risk-gender, i.e. differently regulated in males and females with regard to risk for Diabetes mellitus type 2 (IFG, IGT and IFG&IGT) and controls. Metabolites sorted by p-value. [IFG = Impaired Fasting Glucose; IGT = Impaired Glucose Tolerance; IFG&IGT: patients having both IFG and IGT]**

| | | |
|---|---|---|
| metabolite | reg_male | risk_group |
| tryptophane | down | IGT |
| alanine | down | IFG |
| leucine | down | IGT |
| palmitic acid | down | IFG |
| eicosatrienoic acid | down | IGT |
| glycerophospholipids | | IGT |
| isoleucine | down | IFG |
| eicosatrienoic acid | down | IFG |
| tryptophane | down | IFG |
| lignoceric acid | down | IGT |
| linoleic acid | down | IGT |
| serine | up | IFG |
| tyrosine | down | IGT |
| linoleic acid | down | IFG |
| pregnenolone sulfate | down | IGT |
| aspartate | up | IGT |
| arachidonic acid | down | IGT |
| succinate | up | IFG&IGT |

**Table 21: Metabolites differing specifically between female controls and female patients from risk groups for Diabetes. Metabolites differing significantly (p < 0,05) with regard to interaction risk-gender, i.e. differently regulated in males and females with regard to risk for Diabetes mellitus type 2 (IFG, IGT and IFG&IGT) and controls. Metabolites sorted by p-value. [IFG = Impaired Fasting Glucose; IGT = Impaired Glucose Tolerance; IFG&IGT: patients having both IFG and IGT]**

| metabolite | reg_female | risk_group |
|---|---|---|
| alanine | up | IFG |
| palmitic acid | up | IFG |
| isoleucine | up | IFG |
| eicosatrienoic acid | up | IFG |
| uric acid | up | IFG |
| stearic acid | up | IFG |
| serine | down | IFG |

### Example 2: Correlation of plasma metabolites with cystatin C

In a metabolomics study comprising healthy individuals as well as patients with CHF of different types and severity classified according to NYHA stage and the correlation of metabolites with Cystatin C levels was investigated, using an ANOVAsmall polar metabolites were positively correlated in plasma (Table 22).

**Table 22: Correlation of metabolites with Cystatin C. Shown are metabolites that were positively correlated in human plasma with Cystatin C levels (p-value < 0.05). Further shown is the ratio of that metabolites between healthy individuals as well as patients with CHF of different types and severity classified according to NYHA stage.**

| **METABOLITE_NAME** | **PVALUE** | **RATIO** |
|---|---|---|
| Aldosterone | 0.0000587 | 1.3849 |
| Pentadecenoic acid (C15:cis[10]1) | 0.00484362 | 1.286 |
| TAG_conjugated Linoleic acid (C18:cis[9]trans[11]2) | 0.00274666 | 1.2257 |
| PC_conjugated Linoleic acid (C18:cis[9]trans[11]2) | 0.01436876 | 1.1917 |
| Glycocholic acid | 0.02083947 | 1.1854 |
| FFA_Palmitoleic acid (C16:cis[9]1) | 0.00085376 | 1.179 |
| Sucrose | 0.00169411 | 1.1771 |
| Timolol | 0.00676457 | 1.1751 |
| FFA_conjugated Linoleic acid (C18:cis[9]trans[11]2) | 0.01218157 | 1.1738 |
| 4-Hydroxy-3-methoxymandelic acid | 0.01604282 | 1.1641 |
| 18-Hydroxycorticosterone | 0.02350604 | 1.1573 |
| 1-Methylhistidine | 8.96E-08 | 1.1555 |
| CE_Cholesterylester C14:1 | 0.00162974 | 1.1458 |
| CE_Cholesterylester C12:0 | 0.00915302 | 1.1377 |
| PE_cis-Vaccenic acid | 0.01122019 | 1.1365 |
| TAG_Myristic acid (C14:0) | 0.01858981 | 1.1286 |
| TAG_Palmitoleic acid (C16:cis[9]1) | 0.01751954 | 1.1273 |
| Pseudouridine | 1.01E-21 | 1.1253 |
| Lauric acid (C12:0) | 0.01944424 | 1.1253 |
| Galactonic acid | 0.02827966 | 1.1172 |
| FFA_Myristic acid (C14:0) | 0.00043414 | 1.1166 |
| Glycerol-3-phosphate, polar fraction | 0.00251244 | 1.1153 |
| Lactose | 0.0083945 | 1.1142 |
| TAG (C16:0,C16:1) | 0.00377556 | 1.1138 |
| PI_dihomo-gamma-Linolenic acid (C20:cis[8,11,14]3) | 0.01492842 | 1.1118 |
| TAG_Eicosenoic acid (C20:cis[11]1) | 0.01897445 | 1.1086 |
| Homovanillic acid (HVA) | 0.00049775 | 1.1079 |
| Palmitoleic acid (C16:cis[9]1) | 0.00308213 | 1.1066 |
| Myristic acid (C14:0) | 0.00703577 | 1.1064 |
| Cystine | 8.64E-07 | 1.105 |
| Cellobiose | 0.00904955 | 1.1028 |
| PE_Linoleic acid (C18:cis[9,12]2) | 0.00736073 | 1.1 |
| CE_Cholesterylester C15:0 | 0.0000859 | 1.0994 |
| Melezitose | 0.02701434 | 1.0987 |
| conjugated Linoleic acid (C18:trans[9,11]2) | 0.00034109 | 1.0982 |
| Sphingosine-1-phosphate (d16:1) | 0.0000112 | 1.0966 |
| Erythrol | 5.52E-07 | 1.0956 |
| PC_trans-Vaccenic acid (C18:trans[11]1) | 0.00806179 | 1.0955 |
| Metoprolol | 0.0018019 | 1.0921 |
| Phenacetin | 0.0119848 | 1.0914 |
| PE_Palmitic acid (C16:0) | 0.01111048 | 1.091 |
| Isopalmitic acid (C16:0) | 0.00090355 | 1.0904 |
| Norvaline | 0.01594927 | 1.089 |
| 3-Methoxytyrosine | 0.00046215 | 1.0878 |
| myo-Inositol | 2.15E-09 | 1.0875 |
| CE_Cholesterylester C16:1 | 0.01203778 | 1.0866 |
| PE_Stearic acid (C18:0) | 0.02000861 | 1.0859 |
| TAG_Stearic acid (C18:0) | 0.03708277 | 1.0858 |
| PC_Palmitoleic acid (C16:cis[9]1) | 0.03528904 | 1.0845 |
| TAG_Elaidic acid (C18:trans[9]1) | 0.04166398 | 1.0833 |
| PI_Oleic acid (C18:cis[9]1) | 0.01087038 | 1.0822 |
| Isocitrate | 3.88E-07 | 1.0818 |
| Creatinine | 0.00162734 | 1.0812 |
| Threitol | 0.02038094 | 1.0811 |
| PE_Oleic acid (C18:cis[9]1) | 0.03203527 | 1.0804 |
| TAG_Palmitic acid (C16:0) | 0.03434188 | 1.0803 |
| 14-Methylhexadecanoic acid | 0.00523372 | 1.0796 |
| Urea | 0.00572012 | 1.0795 |
| CER_Ceramide (d16:1,C22:1) | 0.01363344 | 1.0795 |
| Kynurenic acid | 0.00887948 | 1.0791 |
| Proline | 0.00010698 | 1.0783 |
| PI_Palmitic acid (C16:0) | 0.00447938 | 1.078 |
| Sphingosine-1-phosphate (d17:1) | 0.00013659 | 1.0767 |
| Citrulline | 0.0000601 | 1.0766 |
| Glucose, lipid fraction | 0.00880658 | 1.0758 |
| Heptadecenoic acid (C17:cis[10]1) | 0.00384771 | 1.075 |
| PE_Palmitoleic acid (C16:cis[9]1) | 0.01284879 | 1.0747 |
| FFA_Oleic acid (C18:cis[9]1) | 0.04512226 | 1.073 |
| Norleucine | 0.02655041 | 1.0729 |
| PI_Linoleic acid (C18:cis[9,12]2) | 0.03037486 | 1.0714 |
| Ribonic acid | 0.00065116 | 1.0711 |
| alpha-Ketoglutarate | 0.00031263 | 1.0697 |
| Saccharic acid | 0.01058718 | 1.0682 |
| Glucose-1-phosphate | 0.04620231 | 1.0674 |
| CER_Ceramide (d18:2,C22:1) | 0.01177441 | 1.0669 |
| S-Adenosylhomocysteine | 0.00085876 | 1.0668 |
| CER_Ceramide (d18:1,C23:1) | 0.00482234 | 1.0664 |
| PI_cis-Vaccenic acid | 0.03666484 | 1.0663 |
| TAG (C16:0,C18:2) | 0.02954298 | 1.0646 |
| Uric acid | 0.00011566 | 1.0645 |
| Cystathionine | 0.01743588 | 1.0643 |
| PC_Docosapentaenoic acid (C22:cis[7,10,13,16,19]5) | 0.02400337 | 1.0643 |
| myo-Inositol-2-phosphate, lipid fraction | 0.04247546 | 1.0637 |
| trans-4-Hydroxyproline | 0.0380941 | 1.0632 |
| FFA_Palmitic acid (C16:0) | 0.03262711 | 1.0611 |
| Galactitol | 0.00441193 | 1.0609 |
| SM_Sphingomyelin (d18:2,C14:0) | 0.00137019 | 1.0599 |
| CER_Ceramide (d17:1,C16:0) | 0.00481832 | 1.0572 |
| CE_Cholesterylester C16:2 | 0.02663127 | 1.0569 |
| CER_Ceramide (d18:1,C14:0) | 0.00657081 | 1.0563 |
| CER_Ceramide (d18:2,C14:0) | 0.01441601 | 1.0559 |
| MAG_Oleic acid (C18:cis[9]1) | 0.03574089 | 1.0549 |
| SM_Sphingomyelin (d18:1,C14:0) | 0.00027973 | 1.0546 |
| Sphingadienine-1-phosphate (d18:2) | 0.00099076 | 1.0543 |
| Cysteine | 0.00000134 | 1.0541 |
| 7-Methylguanine | 0.02521028 | 1.0537 |
| CE_Cholesterylester C14:0 | 0.00691342 | 1.0536 |
| Eicosenoic acid (C20:cis[11]1) | 0.04645017 | 1.053 |
| Malate | 0.01003198 | 1.0505 |
| PC_dihomo-gamma-Linolenic acid (C20:cis[8,11,14]3) | 0.03194076 | 1.0503 |
| CER_Ceramide (d18:1,C16:0) | 0.00041067 | 1.049 |
| Galactose, lipid fraction | 0.0085052 | 1.0487 |
| CER_Ceramide (d18:1,C22:1) | 0.04844811 | 1.0471 |
| MAG_cis-Vaccenic acid | 0.03095777 | 1.0465 |
| MAG_Palmitoleic acid (C16:cis[9]1) | 0.03531624 | 1.0465 |
| Cholesterol, free | 0.00031928 | 1.046 |
| Citrate | 0.00520095 | 1.0459 |
| CE_Cholesterylester C22:4 | 0.04253297 | 1.0456 |
| CER_Ceramide (d16:1,C16:0) | 0.03631033 | 1.0452 |
| Erythronic acid | 0.00345681 | 1.0449 |
| SM_Sphingomyelin (d17:1,C16:0) | 0.00421295 | 1.0449 |
| Heptadecanoic acid (C17:0) | 0.01706649 | 1.0444 |
| CE_Cholesterylester C20:3 | 0.04978668 | 1.0434 |
| CER_Ceramide (d18:2,C24:2) | 0.0485298 | 1.043 |
| Ornithine | 0.00233216 | 1.0411 |
| Sphingosine-1-phosphate (d18:1) | 0.02652481 | 1.0404 |
| CER_Ceramide (d18:1,C24:2) | 0.0350854 | 1.0401 |
| CER_Ceramide (d18:0,C16:0) | 0.0417336 | 1.0395 |
| CER_Ceramide (d18:2,C16:0) | 0.01328616 | 1.0394 |
| 2-Hydroxypalmitic acid (C16:0) | 0.01357086 | 1.0381 |
| SM_Sphingomyelin (d16:1,C16:0) | 0.02603957 | 1.0375 |
| 3,4-Dihydroxyphenylalanine (DOPA) | 0.03324624 | 1.0365 |
| Arginine | 0.03208162 | 1.0319 |
| SM_Sphingomyelin (d18:1,C23:1) | 0.04107742 | 1.0309 |
| PE_Elaidic acid (C18:trans[9]1) | 0.00830393 | 1.0263 |
| Phenylalanine | 0.01504421 | 1.0262 |
| SM_Sphingomyelin (d18:1,C16:0) | 0.02973833 | 1.026 |
| PE_trans-Vaccenic acid (C18:trans[11]1) | 0.04596375 | 1.0242 |
| SM_Sphingomyelin (d18:0,C16:0) | 0.04518524 | 1.0238 |
| Phosphatidylcholine (C18:0,C18:1) | 0.02490652 | 1.0208 |
| PC_Myristoleic acid (C14:cis[9]1) | 0.00672447 | 1.0093 |
| LPC_Docosatetraenoic acid (C22:cis[7,10,13,16]4) | 0.02863556 | 1.0083 |

### Example 3: Correction of plasma and urine metabolites for renal clearance

In a metabolomics study comprising healthy individuals as well as patients with CHF of different types and severity classified according to NYHA stage. The metabolic profile was analysed as described in anyone of WO2011/092285 A2, WO2012/085890, WO2007/110357 or WO2007/110358. The nomenclature of lipids from the analysis of complex lipids has been applied like described in WO2011/092285. Groups contained individuals with or without reduced renal function. A ANOVA was calculated based on a dataset using the ANOVA model (correcting for age, body mass index, gender, sample storage time, diabetes, see Table 23A, columns headed conf_int_diab) and second the ANOVA model including the additional normalization to cystatin C (see Table 23, columns headed conf_int_diab_CysC). The investigated patient group was classified according to diagnosis as DCMP or HCMP and the severity of the disease was classified according to NYHA.

Individuals with reduced renal function can be determined by significantly (p-value <0.05) increased Urea levels. A detail of the dataset with and without normalization to cystatin C is givenin Table 23. The data were evaluated with and without normalization to cystatin C. We found the classic biomarker for CHF NT-BNP gives higher fold change values. For example: In the contrast subgroup NYHA group DCMP_II-III to control the fold change increased after cystatin C correction from 8.5928 to 9.0259, Table 23A while significance values remained highly significant with p-value of approximately 10E-22, Table23B). Simultaneously, the fold change of the renal kidney disease biomarker urea measured both by metabolite profiling and by conventional techniques became less by the normalization procedure. For example: In the contrast subgroup NYHA group DCMP_II-III to control the fold change of urea values determined in this study decreased after cystatin C correction from 1.103 to 1.0743 Table23A. At the same time the significance of these fold changes was less and p-values increased from 0.054782 to 0.170723, respectively, Table 23B.

These observations are in accordance with the invention described herein. This data demonstrated that normalization by cystatin C values compensates for the distortion of metabolite concentrations introduced by renal dysfunction. While CHF biomarkers show enhanced significance, renal dysfunction biomarkers show less significance.

**Table 23: Detail from data table in CHF study demonstrating the effect of cystatin C normalization on the dataset. While the significance of the CHF biomarker NT-proBNP improves, significance of CKD biomarker Urea decreases. Other potential markers are included in the list. In the DCMP NYHAI group the patients generally have normal kidney function, consequently normalization via cystatin C does not effect the data much.**

| A | | | | | | | | |
|---|---|---|---|---|---|---|---|---|
| | **Without cystatin C normalization** | | | | **With cystatin C normalization** | | | |
| **Model_type** | conf_int _diab | conf_in t_diab | conf_in t_diab | conf_int _diab | conf_int _diab_C ysC | conf_int _diab_C ysC | conf_in t_diab_ CysC | conf_int _diab_ CysC |
| **Factor** | SUBGROUP _NYHA | SUB-GROU P_NY | SUB-GROU P_NY | SUBGROUP _NYHA | SUBGROUP _NYHA | SUBGROUP _NYHA | SUB-GROU P_NY | SUB-GROU P_NYH |

| | GROUP | HAGR OUP | HAGR OUP | GROUP | GROUP | GROUP | HAGR OUP | AG-ROUP |
|---|---|---|---|---|---|---|---|---|
| **Group** | DCMP_I - I/II | DCMP _II - III | HCMP _I - I/II | HCMP_I I - III | DCMP_I - I/II | DCMP_I I - III | HCMP _I - I/II | HCMP_ II - III |
| **References** | control | control | control | control | control | control | control | control |
| **METABOLITE_NAME** | **RATIO** | **RATIO** | **RATIO** | **RATIO** | **RATIO** | **RATIO** | **RATIO** | **RATIO** |
| NT-proBNP | 4,5547 | 8,5928 | 1,6755 | 2,1366 | 5,0965 | 9,0259 | 1,7893 | 2,168 |
| | | | | | | | | |
| Isocitrate | 1,2052 | 1,3852 | 1,0979 | 1,1462 | 1,228 | 1,3162 | 1,1205 | 1,1193 |
| Urea | 1,1463 | 1,1003 | 0,9792 | 1,1093 | 1,1386 | 1,0743 | 0,9847 | 1,0839 |
| SM_Sphingomy elin (d17:1,C24:1) | 0,924 | 0,7919 | 0,9316 | 0,9581 | 0,9094 | 0,7932 | 0,9236 | 0,9349 |
| SM_Sphingomy elin (d17:1,C20:0) | 0,8909 | 0,7285 | 0,9004 | 0,9047 | 0,8525 | 0,7496 | 0,8856 | 0,8848 |
| SM_Sphingomy elin (d18:2,C23:0) | 0,8891 | 0,7666 | 0,8811 | 0,9123 | 0,8619 | 0,7884 | 0,8746 | 0,9005 |
| erythro-C16-Sphingosine | 0,8608 | 0,647 | 0,9202 | 0,9629 | 0,8163 | 0,6174 | 0,9084 | 0,9148 |
| SM_Sphingomy elin (d16:1,C22:0) | 0,8563 | 0,6732 | 0,9299 | 0,9449 | 0,7936 | 0,6629 | 0,914 | 0,9056 |
| Tricosanoic acid (C23:0) | 0,844 | 0,6899 | 0,8573 | 0,8827 | 0,8248 | 0,7003 | 0,8546 | 0,8642 |
| SM_Sphingomy elin (d16:1,C23:0) | 0,834 | 0,6229 | 0,8791 | 0,8449 | 0,7545 | 0,6059 | 0,8654 | 0,808 |
| 1-Hydroxy-2-amino-(cis,trans)-3,5-octadecadiene (from sphingolipids) | 0,831 | 0,6707 | 0,8837 | 0,9224 | 0,8151 | 0,6522 | 0,878 | 0,8853 |
| SM_Sphingomy elin (d17:1,C22:0) | 0,818 | 0,6755 | 0,9113 | 0,9053 | 0,7652 | 0,6692 | 0,8983 | 0,8749 |
| SM_Sphingomy elin (d17:1,C24:0) | 0,795 | 0,6429 | 0,9127 | 0,8534 | 0,7504 | 0,6344 | 0,9046 | 0,8255 |
| SM_Sphingomy elin (d16:1,C24:0) | 0,7652 | 0,5994 | 0,8963 | 0,96 | 0,7354 | 0,5989 | 0,8835 | 0,9171 |
| Cholesterylester C18:2 | 0,749 | 0,6681 | 0,7413 | 0,7695 | 0,7875 | 0,7499 | 0,7041 | 0,7726 |
| SM_Sphingomy elin (d17:1,C23:0) | 0,737 | 0,5707 | 0,8211 | 0,8594 | 0,6987 | 0,5801 | 0,8117 | 0,8216 |
| CE_Cholesteryl ester C15:0 | 0,709 | 0,6213 | 0,8249 | 0,835 | 0,6711 | 0,576 | 0,8448 | 0,7958 |

| B | | | | | | | | |
|---|---|---|---|---|---|---|---|---|
| | **Without cystatin C normalization** | | | | **With cystatin C normalization** | | | |
| **Model_type** | conf_int _diab | conf_in t_diab | conf_in t_diab | conf_int _diab | conf_int _diab_C ysC | conf_int _diab_C ysC | conf_in t_diab_ CysC | conf_int _diab_C ysC |
| **Factor** | SUBGROUP _NYHA GROUP | SUB-GROU P_NY HAGR OUP | SUB-GROU P_NY HAGR OUP | SUBGROUP NYHA GROUP | SUBGROUP _NYHA GROUP | SUBGROUP _NYHA GROUP | SUB-GROU P_NY HAGR OUP | SUBGROUP NYHA GROUP |
| **Group** | DCMP_I - I/II | DCMP _II - III | HCMP _I - I/II | HCMP_I I - III | DCMP_I - I/II | DCMP_I I - III | HCMP _I - I/II | HCMP_I I - III |
| **References** | control | control | control | control | control | control | control | control |
| **METABOLITE_MAME** | **PVA-LUE** | **PVA-LUE** | **PVA-LUE** | **PVA-LUE** | **PVA-LUE** | **PVA-LUE** | **PVA-LUE** | **PVA-LUE** |
| NT-proBNP | 4,82E-12 | 1,74E-22 | 0,0156 078 | 0,00101 68 | 2E-13 | 6,3E-22 | 0,0044 124 | 0,00058 346 |
| Isocitrate | 0,00024 015 | 8,68E-11 | 0,0657 395 | 0,01236 1 | 0,00005 17 | 7,3E-08 | 0,0186 496 | 0,03252 52 |
| Urea | 0,00826 734 | 0,0547 822 | 0,6843 029 | 0,06233 97 | 0,01400 49 | 0,17072 336 | 0,7602 796 | 0,14485 695 |
| SM_Sphingomy elin (d17:1,C24:1) | 0,08192 016 | 3,09E-07 | 0,1099 126 | 0,35533 87 | 0,04905 507 | 0,00000 496 | 0,0760 524 | 0,15704 406 |
| SM_Sphingomy elin (d17:1,C20:0) | 0,03550 351 | 1,04E-08 | 0,0501 579 | 0,07412 93 | 0,00517 297 | 0,00000 15 | 0,0215 779 | 0,02941 14 |
| SM_Sphingomy elin (d18:2,C23:0) | 0,01328 734 | 2,54E-08 | 0,0063 287 | 0,05809 23 | 0,00330 97 | 0,00000 703 | 0,0043 336 | 0,03530 388 |
| erythro-C16-Sphingosine | 0,03161 874 | 3,26E-10 | 0,2348 185 | 0,61444 31 | 0,00611 932 | 2,09E-10 | 0,1751 897 | 0,24915 906 |
| SM_Sphingomy elin (d16:1,C22:0) | 0,01504 927 | 8,45E-10 | 0,2411 024 | 0,38263 39 | 0,00059 425 | 7,33E-09 | 0,1464 397 | 0,13218 534 |
| Tricosanoic acid (C23:0) | 0,00205 816 | 1,17E-11 | 0,0053 604 | 0,03490 24 | 0,00093 78 | 1,84E-09 | 0,0049 405 | 0,01644 277 |
| SM_Sphingomy elin (d16:1,C23:0) | 0,00774 815 | 8,55E-12 | 0,0520 314 | 0,01543 43 | 0,00008 05 | 4,1E-11 | 0,0277 569 | 0,00236 719 |
| 1-Hydroxy-2-amino-(cis,trans)-3,5-octadecadiene (from sphingolipids) | 0,00197 934 | 1,86E-11 | 0,0392 421 | 0,20830 86 | 0,00134 181 | 5,98E-11 | 0,0330 915 | 0,06709 363 |
| SM_Sphingomy elin (d17:1,C22:0) | 0,00029 769 | 3,18E-12 | 0,0838 062 | 0,07721 37 | 0,00000 544 | 1,01E-10 | 0,0464 069 | 0,01971 957 |
| SM_Sphingomy elin (d17:1,C24:0) | 0,00007 13 | 5,84E-14 | 0,1012 501 | 0,00684 72 | 0,00000 457 | 7,46E-12 | 0,0797 326 | 0,00174 465 |
| SM_Sphingomy elin (d16:1,C24:0) | 0,00232 813 | 6,77E-09 | 0,1990 696 | 0,64736 53 | 0,00113 505 | 2,39E-07 | 0,1545 728 | 0,34904 969 |
| Cholesterylester C18:2 | 0,00002 36 | 1,74E-09 | 0,0000 138 | 0,00036 4 | 0,00181 547 | 0,00029 474 | 0,0000 011 | 0,00066 417 |
| SM_Sphingomy elin (d17:1,C23:0) | 0,00030 377 | 4,76E-11 | 0,0161 611 | 0,07683 64 | 0,00006 54 | 8,13E-09 | 0,0116 072 | 0,02521 292 |
| CE_Cholesteryl ester C15:0 | 0,00001 38 | 1,69E-09 | 0,0117 082 | 0,02405 09 | 0,00000 179 | 3,29E-10 | 0,0269 829 | 0,00498 157 |

## Claims

1. A method for determining a clearance normalized amount of a metabolite disease biomarker in one type of sample comprising the steps of:
(a) determining the amount of the metabolite disease biomarker in said type of sample of a subject suspected to suffer from the disease;
(b) determining the amount of a kidney function biomarker which correlates with the glomerular filtration rate (GFR) in the same type of sample; and
(c) determining a clearance normalized amount for the metabolite disease biomarker by normalizing the amount determined for the metabolite disease biomarker in step (a) to the amount of the kidney function biomarker determined in step (b),
wherein said type of sample is blood or a derivative thereof.

2. The method of claim 1, wherein said kidney function biomarker is selected from the group consisting of cystatin C.

3. The method of claim 1 or 2, wherein said metabolite disease biomarker is a biomarker for cardiovascular diseases or disorders, diabetes or metabolic syndrome or neurodegenerative diseases.

4. The method of any one of claims 1 to 3, wherein said metabolite disease biomarker is a biomarker selected from any of tables 1 to 6 and 8 to 21.

5. The method of any one of claims 1 to 4, wherein said normalizing in step (c) encompasses calculating a ratio of the amount determined for the metabolite disease biomarker in step (a) and the amount of the kidney function biomarker determined in step (b).

6. The method of any one of claims 1 to 5, wherein steps (a) and (b) are carried out on the same aliquot of a sample of said type, on different aliquots of a sample of said type, or on aliquots of different samples of said type.

7. A method for diagnosing a disease in a subject suspected to suffer therefrom comprising:
(a) determining a clearance normalized amount for a metabolite disease biomarker in a sample of said subject according to the method of any one of claims 1 to 6; and
(b) comparing said clearance normalized amount to a reference, whereby the disease is to be diagnosed.

8. The method of claim 7, wherein said disease is a cardiovascular diseases or disorders, diabetes or metabolic syndrome or neurodegenerative diseases.

9. The method of claim 7 or 8, wherein said metabolite disease biomarker is a biomarker selected from any of tables 1 to 6 and 8 to 21.

10. Use of a kidney function biomarker in a sample of a subject comprising a metabolite disease biomarker for normalizing said metabolite disease biomarker, wherein said sample is blood or a derivative thereof.

11. The use of claim 10, wherein said kidney function biomarker is selected from the group consisting of cystatin C.

12. A device for determining a clearance normalized amount of a metabolite disease biomarker in a sample according to the method of any one of claims 1 to 6, said device comprising:
a) an analyzing unit comprising a detection agent which specifically detects the amount of at least one metabolite disease biomarker in at least a first type of sample and a detection agent which specifically detects the amount of a kidney function biomarker in said at least first type of sample; and
b) an evaluation unit comprising a data processor having tangibly embedded a computer program code carrying out an algorithm which normalizes the amount for the metabolite disease biomarker to the amount of the kidney function biomarker,
wherein said normalization encompasses calculating a ratio of the amount determined for the metabolite disease biomarker and the amount of the kidney function biomarker.

13. The device of claim 12, wherein said evaluation unit comprises a database with stored references which allow for diagnosing a disease based on the clearance normalized amount for the metabolite disease biomarker.

## Patentansprüche

1. Verfahren zum Bestimmen einer Clearance-normalisierten Menge eines Stoffwechselkrankheitsbiomarkers in einem Typ einer Probe, umfassend die folgenden Schritte:
(a) Bestimmen der Menge des Stoffwechselkrankheitsbiomarkers in dem Typ einer Probe eines Individuums, bei dem der Verdacht besteht, dass es an der Krankheit leidet;
(b) Bestimmen der Menge eines Nierenfunktionsbiomarkers, der mit der Glomerulifiltrationsrate (GFR) in demselben Typ von Probe korreliert; und
(c) Bestimmen einer Clearance-normalisierten Menge für den Stoffwechselkrankheitsbiomarker durch Normalisieren der für den Stoffwechselkrankheitsbiomarker in Schritt (a) bestimmten Menge gegenüber der für den Stoffwechselkrankheitsbiomarker in Schritt (b) bestimmten Menge,
wobei es sich bei dem Typ von Probe um Blut oder ein Derivat davon handelt.

2. Verfahren nach Anspruch 1, wobei der Nierenfunktionsbiomarker aus der Gruppe bestehend aus Cystatin C ausgewählt ist.

3. Verfahren nach Anspruch 1 oder 2, wobei der Stoffwechselkrankheitsbiomarker ein Biomarker für Herz-Kreislauf-Krankheiten oder -Störungen, Diabetes oder metabolisches Syndrom oder neurodegenerative Krankheiten ist.

4. Verfahren nach einem der Ansprüche 1 bis 3, wobei der Stoffwechselkrankheitsbiomarker ein Biomarker, ausgewählt aus einer der Tabellen 1 bis 6 und 8 bis 21, ist.

5. Verfahren nach einem der Ansprüche 1 bis 4, wobei das Normalisieren in Schritt (c) das Berechnen eines Verhältnisses der für den Stoffwechselkrankheitsbiomarker in Schritt (a) bestimmten Menge und der für den Nierenfunktionsbiomarker in Schritt (b) bestimmten Menge umfasst.

6. Verfahren nach einem der Ansprüche 1 bis 5, wobei die Schritte (a) und (b) an demselben Aliquot einer Probe dieses Typs, an unterschiedlichen Aliquots einer Probe dieses Typs oder an Aliquots von unterschiedlichen Proben dieses Typs durchgeführt werden.

7. Verfahren zum Diagnostizieren einer Krankheit in einem Individuum, bei dem der Verdacht besteht, dass es daran leidet, umfassend die folgenden Schritte:
(a) Bestimmen einer Clearance-normalisierten Menge für einen Stoffwechselkrankheitsbiomarker in einer Probe des Individuums nach dem Verfahren nach einem der Ansprüche 1 bis 6;
(b) Vergleichen der Clearance-normalisierten Menge mit einer Referenz, wodurch die Krankheit diagnostiziert werden soll.

8. Verfahren nach Anspruch 7, wobei die Krankheit eine Herz-Kreislauf-Krankheiten oder -Störungen, Diabetes oder metabolisches Syndrom oder neurodegenerative Krankheiten ist.

9. Verfahren nach Anspruch 7 oder 8, wobei der Stoffwechselkrankheitsbiomarker ein Biomarker, ausgewählt aus einer der Tabellen 1 bis 6 und 8 bis 21, ist.

10. Verwendung eines Nierenfunktionsbiomarkers in einer Probe eines Individuums umfassend einen Stoffwechselkrankheitsbiomarker zum Normalisieren des Stoffwechselkrankheitsbiomarkers, wobei es sich bei der Probe um Blut oder ein Derivat davon handelt.

11. Verwendung nach Anspruch 10, wobei der Nierenfunktionsbiomarker aus der Gruppe bestehend aus Cystatin C ausgewählt ist.

12. Vorrichtung zum Bestimmen einer Clearance-normalisierten Menge eines Stoffwechselkrankheitsbiomarkers in einer Probe nach dem Verfahren nach einem der Ansprüche 1 bis 6, wobei die Vorrichtung Folgendes umfasst:
a) eine Analyseeinheit umfassend ein Nachweismittel, das spezifisch die Menge an mindestens einem Stoffwechselkrankheitsbiomarker in mindestens einem ersten Typ von Probe nachweist, und ein Nachweismittel, das spezifisch die Menge eines Nierenfunktionsbiomarkers in dem mindestens ersten Typ von Probe nachweist; und
b) eine Auswertungseinheit umfassend einen Rechner mit einem greifbar eingebetteten Computerprogrammcode zur Durchführung eines Algorithmus, der die Menge für den Stoffwechselkrankheitsbiomarker in Bezug auf die Menge des Nierenfunktionsbiomarkers normalisiert,
wobei das Normalisieren das Berechnen eines Verhältnisses der für den Stoffwechselkrankheitsbiomarker bestimmten Menge und der Menge des Nierenfunktionsbiomarkers umfasst.

13. Vorrichtung nach Anspruch 12, wobei die Auswertungseinheit eine Datenbank mit gespeicherten Referenzen umfasst, die das Diagnostizieren einer Krankheit auf Grundlage der Clearance-normalisierten Menge für den Stoffwechselkrankheitsbiomarker gestattet.

## Revendications

1. Procédé de détermination d'une quantité normalisée pour la clairance d'un biomarqueur de maladie métabolique dans un type d'échantillon comprenant les étapes de :
(a) détermination de la quantité du biomarqueur de maladie métabolique dans ledit type d'échantillon d'un sujet suspecté de souffrir de la maladie ;
(b) détermination de la quantité d'un biomarqueur de fonction rénale qui est corrélée avec le débit de filtration glomérulaire (DFG) dans le même type d'échantillon ; et
(c) la détermination d'une quantité normalisée pour la clairance du biomarqueur de maladie métabolique par normalisation de la quantité déterminée pour le biomarqueur de maladie métabolique dans l'étape (a) par rapport à la quantité du biomarqueur de fonction rénale déterminée dans l'étape (b),
dans lequel ledit type d'échantillon est du sang ou un dérivé de celui-ci.

2. Procédé de la revendication 1, dans lequel ledit biomarqueur de fonction rénale est choisi dans le groupe constitué de la cystatine C.

3. Procédé de la revendication 1 ou 2, dans lequel ledit biomarqueur de maladie métabolique est un biomarqueur de maladies ou troubles cardiovasculaires, du diabète ou du syndrome métabolique ou de maladies neurodégénératives.

4. Procédé de l'une quelconque des revendications 1 à 3, dans lequel ledit biomarqueur de maladie métabolique est un biomarqueur choisi parmi l'un quelconque des tableaux 1 à 6 et 8 à 21.

5. Procédé de l'une quelconque des revendications 1 à 4, dans lequel ladite normalisation dans l'étape (c) comprend le calcul d'un rapport de la quantité déterminée pour le biomarqueur de maladie métabolique dans l'étape (a) et la quantité du biomarqueur de fonction rénale déterminée dans l'étape (b) .

6. Procédé de l'une quelconque des revendications 1 à 5, dans lequel les étapes (a) et (b) sont conduites sur la même aliquote d'un échantillon dudit type, sur différentes aliquotes d'un échantillon dudit type, ou sur des aliquotes de différents échantillons dudit type.

7. Procédé de diagnostic d'une maladie chez un sujet suspecté de souffrir de celle-ci comprenant :
(a) la détermination d'une quantité normalisée pour la clairance pour un biomarqueur de maladie métabolique dans un échantillon dudit sujet selon le procédé de l'une quelconque des revendications 1 à 6 ; et
(b) la comparaison de ladite quantité normalisée pour la clairance à une référence, afin que la maladie soit diagnostiquée.

8. Procédé de la revendication 7, dans lequel ladite maladie est choisie parmi un maladies ou troubles cardiovasculaires, le diabète ou le syndrome métabolique ou des maladies neurodégénératives.

9. Procédé de la revendication 7 ou 8, dans lequel ledit biomarqueur de maladie métabolique est un biomarqueur choisi parmi l'un quelconque des tableaux 1 à 6 et 8 à 21.

10. Utilisation d'un biomarqueur de fonction rénale dans un échantillon d'un sujet comprenant un biomarqueur de maladie métabolique pour normaliser ledit biomarqueur de maladie métabolique, dans laquelle ledit échantillon est du sang ou un dérivé de celui-ci.

11. Utilisation de la revendication 10, dans laquelle ledit biomarqueur de fonction rénale est choisi dans le groupe constitué de la cystatine C.

12. Dispositif de détermination d'une quantité normalisée pour la clairance d'un biomarqueur de maladie métabolique dans un échantillon selon le procédé de l'une quelconque des revendications 1 à 6, ledit dispositif comprenant :
a) une unité d'analyse comprenant un agent de détection qui détecte spécifiquement la quantité d'au moins un biomarqueur de maladie métabolique dans au moins un premier type d'échantillon et un agent de détection qui détecte spécifiquement la quantité d'un biomarqueur de fonction rénale dans ledit au moins premier type d'échantillon ; et
b) une unité d'évaluation comprenant un processeur de données dans lequel est incorporé de façon tangible un code de programme informatique exécutant un algorithme qui normalise la quantité pour le biomarqueur de maladie métabolique par rapport à la quantité du biomarqueur de fonction rénale,
dans lequel ladite normalisation comprend le calcul d'un rapport de la quantité déterminée pour le biomarqueur de maladie métabolique et de la quantité du biomarqueur de fonction rénale.

13. Dispositif de la revendication 12, dans lequel ladite unité d'évaluation comprend une base de données avec des références stockées qui permettent le diagnostic d'une maladie sur la base de la quantité normalisée pour la clairance du biomarqueur de maladie métabolique.
